# FASCICULE DE BREVET EUROPEEN

(11) **EP 0 934 290 B1**
(45) Date de publication et mention de la délivrance du brevet: **17.04.2002**
(21) Numéro de dépôt: 97944937.8
(22) Date de dépôt: 07.10.1997
(51) Int. Cl.: C07D 277/42, C07D 417/12, A61K 31/425

(54) **DERIVES D'AMINOTHIAZOLE, LEUR PROCEDE DE PREPARATION ET LES COMPOSITIONS PHARMACEUTIQUES LES CONTENANT**
AMINOTHIAZOLE DERIVATE, IHRE HERSTELLUNG UND SIE ENTHALTENDEN PHARMAZEUTISCHEN ZUSAMMENSETZUNGEN
AMINOTHIAZOLE DERIVATIVES, METHOD OF PREPARATION AND PHARMACEUTICAL COMPOSITIONS CONTAINING SAME

(30) Priorité: 08.10.1996 FR 9612256
(43) Date de publication de la demande: 11.08.1999
(73) Titulaire: SANOFI-SYNTHELABO, 75635 Paris Cédex 13 (FR)
(72) Inventeur: FONTAINE, Evelyne, F-31320 Castanet-Tolosan (FR); GULLY, Danielle, F-31600 Muret (FR); ROGER, Pierre, F-78423 Montigny le Bretonneux (FR); WERMUTH, Camille, Georges, F-67100 Strasbourg (FR)
(74) Mandataire: Bernasconi, Jean Raymond
(86) Numéro de dépôt international: FR9701788
(87) Numéro de publication internationale: WO9815543

(56) Documents cités:
- EP-A- 0 283 390
- EP-A- 0 576 350
- EP-A- 0 659 747
- WO-A-91/09857
- WO-A-94/01423
- WO-A-97/00868

## Description

La présente invention a pour objet de nouveaux dérivés amino du thiazole, un procédé pour leur préparation et les compositions pharmaceutiques les contenant. Ces nouveaux dérivés du thiazole sont pourvus d'activité antagoniste du CRF (corticotropin releasing factor) et peuvent donc constituer des principes actifs de compositions pharmaceutiques.

Le facteur de libération de l'hormone corticotrope (CRF) est un peptide dont la séquence de 41 acides aminés, a été caractérisée par Vale W. *et al.* en 1981 (Science, 1981, *213*, 1394-1397). Le CRF est le principal facteur endogène impliqué dans la régulation de l'axe hypothalamo-hypophyso-surrénalien (libération de l'hormone adrénocorticotrope : ACTH) et ses pathologies, ainsi que dans les syndromes dépressifs qui en découlent. Le CRF provoque également la sécrétion de β-endorphine, de β-lipotropine et de corticostérone. Le CRF est donc le régulateur physiologique de la sécrétion de l'hormone adrénocorticotrope (ACTH) et plus généralement des peptides dérivés de la proopiomélanocortine (POMC). Outre sa localisation hypothalamique, le CRF est largement distribué dans le système nerveux central, mais également dans des tissus extra-neuronaux tels que les glandes surrénales et les testicules. La présence de CRF a été également mise en évidence au cours de processus inflammatoires.

De nombreuses expérimentations animales ont montré que l'administration centrale de CRF provoque des effets anxiogènes variés tels que la modification du comportement en général : par exemple néophobie, réduction de la réceptivité sexuelle, diminution de la consommation alimentaire et du sommeil lent chez le rat. L'injection intracérébroventriculaire de CRF augmente également l'excitation. des neurones noradrénergiques du locus coeruleus qui est souvent associée chez l'animal, à un état d'anxiété. Chez le rat, l'administration centrale ou périphérique du CRF ou des peptides apparentés (par exemple urocortine, sauvagine) induit outre des effets centraux tels que l'augmentation de l'éveil et de la réactivité émotionnelle face à l'environnement, des modifications de la vidange gastrique, de la sécrétion acide, du transit intestinal et de l'excrétion fécale ainsi que des effets tensionnels. Le CRF est impliqué également dans la régulation complexe des réponses inflammatoires, d'une part avec un rôle pro-inflammatoire dans certains modèles animaux, d'autre part en tant qu'inhibiteur des effets induits par l'augmentation de la perméabilité vasculaire consécutive de l'inflammation.

L'utilisation d'un antagoniste peptidique, l'alpha-hélical CRF(9-41) (ah-CRF) ou d'anticorps spécifiques (Rivier J. *et al*., Science, 1984, *224*, 889-891) a permis de confirmer le rôle de ce peptide dans l'ensemble de ces effets. Ces expériences ont également confirmé le rôle important du CRF chez l'homme dans l'intégration des réponses complexes observées lors d'un stress physiologique. psychologique ou immunologique tant sur le plan neuroendocrinien, viscéral que comportemental (Morley J.E. *et al*, Endocrine Review, 1987, *8*, 3, 256-287 ; Smith M.A. *et al*., Horm. Res.. 1989, *31*, 66-71). En outre, des données cliniques militent en faveur de l'implication effective du CRF dans les nombreux désordres découlant d'un état de stress (Gulley L.R. *et al.*, J. Clin. Psychiatry, 1993, *54*, 1, (suppl.), 16-19) par exemple :
- l'existence du test au CRF (administration i.v.) chez l'homme a permis de démontrer la modification de la réponse en ACTH chez les patients dépressifs (Breier A. *et al*., Am. J. Psychiatry, 1987, *144*, 1419-1425).
- la découverte d'une hypersécrétion de CRF endogène dans certaines pathologies, par exemple d'un taux de CRF élevé dans le liquide céphalorachidien chez les patients non médiqués, déprimés ou atteints de démence type maladie d'Alzheimer (Nemeroff C.B. *et al*., Science, 1984, *226*, 4680, 1342-1343 ; Regul. Pept., 1989, *25*, 123-130), ou d'une densité de récepteurs au CRF diminuée dans le cortex de victimes de suicide (Nemeroff C.B. *et al*., Arch. Gen. Psychiatry, 1988, *45*, 577-579).
- le dysfonctionnement des neurones CRF-dépendants est même suggéré dans les pathologies sévères que sont les maladies d'Alzheimer, de Parkinson, la chorée de Huntington et la sclérose latérale amyotrophique (De Souza E.B., Hospital Practice, 1988, *23*, 59).

L'administration centrale de CRF dans de nombreuses espèces animales, produit des effets comportementaux semblables à ceux obtenus chez l'homme dans les situations de stress. Lorsqu'ils sont répétés dans le temps, ces effets peuvent entraîner des pathologies diverses telles que : fatigue, hypertension, troubles cardiaques, modification de la vidange gastrique, de l'excrétion fécale (colite, colon irritable), modification de la sécrétion acide, hyperglycémie, croissance retardée, anorexie, néophobie, troubles de la reproduction, immunosuppression (processus inflammatoires, infections multiples et cancers) et désordres neuropsychiatriques variés (dépression, anorexie nerveuse et anxiété).

L'injection par voie intracérébroventriculaire de l'antagoniste peptidique de référence, l'ah-CRF prévient les effets obtenus soit par l'administration de CRF exogène, soit par l'utilisation d'agents stressants (éther, contrainte, bruit, choc électrique, sevrage éthanolique, chirurgie) capables par eux-mêmes d'induire une augmentation du taux de CRF endogène. Ces résultats sont confirmés par l'étude de nombreuses molécules peptidiques antagonistes structuralement apparentées au CRF et qui possèdent une durée d'action prolongée par rapport à l'ah-CRF (Rivier J. *et al.*, J. Med. Chem.. 1993, *36,* 2851-2859 : Menzaghi F. *et al*., J. Pharmacol. Exp. Ther., 1994, *269,* 2, 564-572, Hernandez J.F. *et al*., J. Med. Chem., 1993. *36,* 2860-2867).

De tels composés peptidiques antagonistes du CRF sont décrits par exemple dans les brevets US 5,109,111, US 5,132,111, US 5,245,009 et dans les demandes de brevet WO 92 22576 et WO 96 19499.

En outre, des études préliminaires ont montré que des antidépresseurs tricycliques pouvaient moduler le taux de CRF ainsi que le nombre de récepteurs au CRF dans le cerveau (Grigoriadis D.E. *et al*., Neuropsychopharmacoloy, 1989, *2,* 53-60). De même des anxiolytiques benzodiazépiniques sont capables d'inverser l'effet du CRF (Britton K.T. *et al*., Psychopharmacology, 1988, *94*, 306), sans que le mécanisme d'action de ces substances soit totalement élucidé. Ces résultats confortent si nécessaire le besoin grandissant de molécules antagonistes non-peptidiques des récepteurs du CRF.

Il est important de signaler également, trois conséquences possibles des états de stress chronique que sont l'immunodépression, les troubles de la fertilité, ainsi que l'installation du diabète.

Le CRF exerce de tels effets en interagissant avec des récepteurs membranaires spécifiques qui ont été caractérisés dans l'hypophyse et le cerveau de nombreuses espèces (souris, rat et homme) ainsi que dans le coeur, le muscle squelettique (rat, souris) et dans le myomètre et le placenta au cours de la grossesse.

Un grand nombre de dérivés du 2-aminothiazole est déjà connu. La demande de brevet EP 462 264 décrit des dérivés du 2-aminothiazole, dont l'amine tertiaire en position 2, comporte deux substituants ayant chacun au moins un hétéroatome dont un dérivé d'amine. Ces composés sont des antagonistes du facteur d'activation.des plaquettes (PAF-acéther) et trouvent leurs applications dans le traitement de l'asthme, de certains états allergiques ou inflammatoires, de maladies cardiovasculaires, de l'hypertension et de diverses pathologies rénales ou encore comme agents contraceptifs.

La demande GB 2 022 285 décrit des composés possédant une activité régulatrice de la réponse immunitaire et ayant des propriétés anti-inflammatoires. Il s'agit de dérivés du thiazole substitués en position 2 par des groupes amines secondaires.

Certains dérivés du 2-acylaminothiazole ont été décrits dans la demande de brevet EP 432 040. Ces composés sont des antagonistes de la cholécystokinine et de la gastrine.

Des dérivés du 2-amino-4,5-diphénylthiazole ayant des propriétés antiinflammatoires sont aussi connus (demande de brevet JP-01 75 475).

On connaît aussi aes dérivés du 2-amino-4-(4-hydroxyphényl)thiazole utiles comme intermédiaires de synthèse pour la préparation des dérivés du 2,2-diarylchroménothiazole (demande de brevet EP 205 069).

Des dérivés du 2-(N-méthyl-N-benzylamino)thiazole sont aussi décrits dans J. Chem. Soc. Perkin, Trans 1, 1984, *2*, 147-153 et dans J. Chem. Soc. Perkin, Trans 1, 1983, *2*, 341-347.

La demande de brevet WO 94 01423 décrit des dérivés du 2-aminothiazole de formule générale : 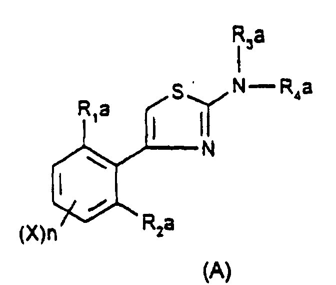 avec R₃a pouvant représenter un alkyle et R₄a un phényle substitué. Ces composés utilisés comme insecticide ne présentent aucune substitution en position 5 de l'hétérocycle.

De même, la demande de brevet WO 96 16650 décrit des composés de formule générale : 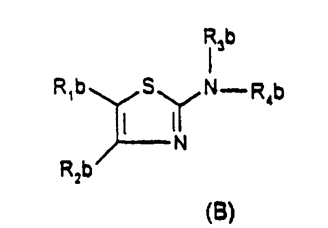 dans lesquels R₂b peut représenter un phényle substitué, R₁b un alkyle, R₃b un alkyle et R₄b un sulfonyle ou un acyle ; ces composés sont utilisés comme antibiotique.

La demande de brevet EP 283 390, décrit parmi d'autres dérivés du thiazole, des dérivés du 2-(N-alkyl-N-pyridylalkylamino)thiazole de formule : 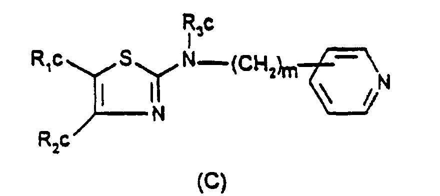 pour lesquels m est différent de zéro.

Ces dérivés, dont l'amine en position 2 est substituée par un radical pyridylalkyle non ramifié, possèdent notamment une activité stimulante de la transmission cholinergique centrale. lls peuvent donc être utilisés comme agonistes des récepteurs muscariniques et trouvent leurs applications dans le traitement des troubles de la mémoire et des démences séniles.

Des dérivés du 2-aminothiazole dont l'amine en position 2 est une amine tertiaire ayant un substituant alkyle ou aralkyle ramifié ont été décrits dans EP 576 350 et dans EP 659. 747 comme possédant une affinité pour les récepteurs du CRF. Aucun de ces composés ne présentent comme substituant de l'amine tertiaire en position 2 du noyau thiazole un phényle substitué.

Le brevet US 5,063,245 décrit des antagonistes du CRF qui permettent de déplacer *in vitro* la liaison du CRF à ses récepteurs spécifiques à une concentration voisine d'une micromole. Depuis de nombreuses demandes de brevets concernant des molécules non-peptidiques ont été publiées, par exemple les demandes WO 94/13643, WO 94/13644, WO 94²13661, WO 94/13676, WO 94/13677, WO 94/10333, WO 95/00640, WO 95/10506, WO 95/13372, WO 95/33727, WO 95/33750, WO 95/34563, EP 691 128 ou EP 729 758.

Il a maintenant été trouvé que certains dérivés amino ramifiés du thiazole, objet de la présente invention, présentent une excellente affinité vis à vis des récepteurs du CRF. De plus, compte tenu de leur structure, ces molécules possèdent une bonne dispersibilité et/ou solubilité dans des solvants ou solutions couramment utilisés en thérapeutique qui leur confère une activité pharmacologique'et permettent aussi la préparation aisée de formes galéniques orales et parentérales.

La présente invention a pour objet, les composés de formule : 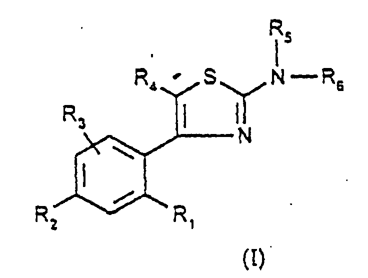 dans laquelle
- R₁ et R₂ identiques ou différents représentent chacun indépendamment un atome d'halogène ; un (C₁-C₅)hydroxyalkyle ; un (C₁-C₅) alkyle ; un aralkyle dans lequel la partie aryle est en (C₆-C₈) et la partie alkyle en (C₁-C₄) ; un (C₁-C₅)alcoxy ; un groupe trifluorométhyle ; un groupe nitro ; un groupe nitrile ; un groupe -SR dans lequel R représente l'hydrogène, un (C₁-C₅)alkyle ou un aralkyle dans lequel la partie aryle est en (C₆-C₈) et la partie alkyle en (C₁-C₄) ; un groupe -S-CO-R^{o} dans lequel R^{o} représente un radical (C₁-C₅)alkyle ou un aralkyle dans lequel la partie aryle est en (C₆-C₈) et la partie alkyle est en (C₁-C₄) ; un groupe -COORa dans lequel Ra représente l'hydrogène ou un (C₁-C₅)alkyle ; un groupe -CONRaRb avec Ra et Rb tels que definis ci-dessus pour Ra ; un groupe -NRaRb avec Ra et Rb tels que définis précédemment pour Ra ; un groupe -CONRcRd ou -NRcRd dans lesquels Rc et Rd constituent avec l'atome d'azote auquel ils sont liés un hétérocycle de 5 à 7 chaînons ; ou un groupe -NHCO-NRaRb avec Ra et Rb tels que définis ci-dessus pour Ra ;
- R₃ représente l'hydrogène ou est tel que défini ci-dessus pour R₁ et R₂ ;
- R₄ représente un (C₁-C₅)alkyle ; un groupe hydroxyméthyle ; un groupe formyle ; ou un atome d'halogène ;
- R₅ représente un (C₁-C₅)alkyle ; un groupe cycloalkylalkyle dans lequel le cycloalkyle est en (C₃-C₇) et l'alkyle en (C₁-C₅) ; un alcényle de 3 à 6 atomes de carbone ; un (C₁-C₅) hydroxyalkyle ; un groupe alkylcarbonyloxyalkyle dans lequel les alkyles sont en (C₁-C₅) ; ou un groupe alcynyle de 3 à 6 atomes de carbone ;
- R₆ représente un phényle substitué par un ou plusieurs substituants Z tel que défini ci-dessous; un groupe hétéroaromatique monocyclique de 5 à 7 chaînons substitué par un ou plusieurs radicaux Z tels que définis ci-dessous; ou un groupe bicyclique de 9 ou 10 chaînons constitué d'un monocycle aromatique comportant éventuellement un ou plusieurs hétéroatomes choisis parmi O, N et S, condensé à un groupe cycloalkyle comportant éventuellement dans le cycle un ou plusieurs hétéroatomes choisis parmi O, N et S, lequel groupe bicyclique est substitué par un ou plusieurs substituants Z tels que définis ci-dessous et lequel est relié à l'azote par le cycle à caractère aromatique, étant entendu que R₆ ne représente pas un indane substitué (de tels composés sont décrits dans la demande de brevet WO 97 00868) et que le substituant Z représente un radical choisi parmi : un atome d'halogène, un groupe nitro, un groupe hydroxy, un groupe tifluorométhyle, un (C₁-C₅)alkyle, un (C₁-C₅)thioalkyle, un groupe -NRaRb avec Ra et Rb tels que définis précédemment pour Ra, un (C₁-C₅)hydroxyalkyle, un (C₁-C₅)alcoxy, un groupe trifluorométhyloxy, un alcoxyalkyle dans lequel les alkyles sont en (C₁-C₅), un groupe -COORa avec Ra tel que défini précédemment, un groupe -CONRaRb avec Ra et Rb tels que définis précédemment pour Ra, un carboxyl(C₁-C₅)alkyle, un alcoxycarbonylalkyle dans lequel les alkyles sont en (C₁-C₅), un (C₁-C₅)alkylcarbonyle, un alkylcarbonylalkyle dans lequel les alkyles sont en (C₁-C₅), un groupe morpholinocarbonyle ou morpholinocarbonyl(C₁-C₅)alkyle, ou un groupe -NRaCOORb avec Ra et Rb tels que définis précédemment, un groupe -NHCORₑ dans lequel Rₑ représente un (C₁-C₈)alkyle, un cycloalkylcarbonyle dans lequel le cycloalkyle est en (C₃-C₆) ; un cycloalkylalkylcarbonyle dans lequel le cycloalkyle est en (C₃-C₅) et l'alkyle en (C₁-C₃), un benzoyle, un phényle non substitué ou substitué par un (C₁-C₅)alkyle, par un (C₁-C₅)alcoxy, par un atome d'halogène, par un groupe nitro, par un groupe hydroxy ou par un groupe trifluorométhyle;
leurs stéréoisomères, leurs sels d'addition, leurs hydrates et/ou leurs solvates.

Par groupe hétéroaromatique monocyclique, on entend particulièrement un groupe choisi parmi azépinyle, pyridyle, pyrazinyle, pyridazinyle, pynmidyle, triazinyle, furyle et thiényle.

Des exemples de groupes bicycliques de 9 ou 10 chaînons sont le 1,2,3,4-tétrahydronaphtyle ainsi que des groupes comportant un ou plusieurs hétéroatomes choisis parmi N. O et S et représentés par exemple par 1,2,3,4-tétrahydroquinolyle, 1,2,3,4-tétrahydroisoquinolyle, 1,2,3,4-tétrahydrophtalazinyle, 1,2,3,4-tétrahydroquinazolinyle, 1,2,3,4-tétrahydroquinoxalinyle, 1,2,3,4-tétrahydrocinnolinyle, 1,2,3,4-tétrahydrobenzo-triazinyle, chromanyle, isochromanyle, indolinyle, isoindolinyle, 2,3-dihydroindazyle, 2,3-dihydrobenzoimidazolyle, 1.2-dihydrobenzotriazolyle, 2,3-dihydrobenzofurannyle, 2,3-dihydrobenzothiényle, 2.3-dihydrobenzoisothiazolyle, 2,3-dihydrobenzothiazolyle, 2,3-dihydrobenzoisoxazolyle, 2,3-dihydrobenzoxazolyle, 1,2-dihydrobenzoxazinyle, 1,2,3,4-tétrahydropténdinyle, 8,9-dihydropurinyle. Ces groupes bicycliques sont substitués par un ou plusieurs substituants Z tels que définis ci-dessus.

Des exemples d'hétérocycles de 5 à 7 chaînons sont la morpholine, la pipéridine ou la pyrrolidine.

Dans la présente description les groupes alkyles ou les groupes alcoxy sont linéaires ou ramifiés.

Par atome d'halogène, on entend atome de fluor, de chlore, de brome ou d'iode.

Des composés avantageux selon l'invention sont ceux dans lesquels R₆ représente un groupe phényle ou tétrahydronaphtyle substitué par un ou plusieurs substituants Z tels que définis pour (I), R₁, R₂, R₃, R₄ et R₅ étant également tels que définis pour (I), un de leurs stéréoisomères, un de leurs sels, un de leurs hydrates et/ou un de leurs solvates.

Parmi ces composés, particulièrement préférés sont les composés dans lesquels R₄ représente un méthyle, R₁, R₂, R₅ et R₃ étant tels que définis pour (I), un de leurs stéréoisomères, un de leurs hydrates et/ou un de leurs solvates.

Plus particulièrement préférés parmi ces composés sont ceux dans lesquels R₁ et/ou R₂ représente un halogène, un trifluorométhyle, un (C₁-C₅)alkyle ou un (C₁-C₅)alcoxy, R₄ représente un méthyle, R₆ représente un phényle au moins substitué en position 2 par un substituant Z tel que défini pour (I). R₃ et R₅ sont tels que définis pour (I), un de leurs stéréoisomères, un de leurs sels, un de leurs hydrates et/ou un de leurs solvates.

Tout particulièrement préférés sont ainsi les composés :
. 4-(2-Chloro-4-méthoxyphényl)-5-mèthyl-2-[N-(5-éthoxycarbonyl-2-méthoxyphényl)-N-propylamino]thiazole
. 4-(2-Chloro-4-méthoxyphényl)-5-méthyl-2-[N-(5-chloro-2-méthylphényl)-N-propyl-amino]thiazole
. 4-(2-Chloro-4-méthoxyphényl)-5-méthyl-2-[N-(2-trifluorométhylphényl)-N-propyl-amino]thiazole
. 4-(2-Chloro-4-méthoxyphényl)-5-méthyl-2-[N-(2-méthoxyphényl)-N-propylamino] thiazole
. 4-(2-Chloro-4-méthoxyphényl)-5-méthyl-2-[N-(2,6-dichlorophényl)-N-propylamino] thiazole
. 4-(2-Chloro-4-méthoxyphényl)-5-méthyl-2-[N-(2,5-dichlorophényl)-N-propylamino] thiazole
. 4-(2-Chloro-4-méthoxyphényl)-5-méthyl-2-[N-(2-chloro-5-méthylphényl)-N-propyl-amino]thiazole
. 4-(2-Chloro-4-méthoxyphényl)-5-méthyl-2-[N-(2-chloro-5-trifluorométhylphényl) -N-propylamino]thiazole
. 4-(2-Chloro-4-méthoxyphényl)-5-méthyl-2-[N-(2-chloro-5-méthoxyphényl)-N -propylamino]thiazole
. 4-(2-Chloro-4-méthoxyphényl)-5-méthyl-2-[N-(5-chloro-2-méthoxyphényl)-N -propylamino]thiazole
. 4-(2-Chloro-4-méthoxyphényl)-5-méthyl-2-[N-(5-chloro-2-éthoxyphényl)-N-propylamino]thiazole
. 4-(2-Chloro-4-méthoxyphényl)-5-méthyl-2-[N-(2-méthoxy-5-méthylphényl)-N -propylamino]thiazole
. 4-(2-Chloro-4-méthoxyphényl)-5-méthyl-2-[N-(2,5-diméthylphényl)-N -propylamino]thiazole
. 4-(2-Chloro-4-méthoxyphényl)-5-méthyl-2-(N-(2,5-ditrifluorométhylphényl)-N -propylamino]thiazole
. 4-(2-Chloro-4-méthoxyphényl)-5-méthyl-2-[N-(2-méthoxy-5-trifluorométhylphényl) -N-propylamino]thiazole
. 4-(2-Chloro-4-méthoxyphényl)-5-méthyl-2-[N-(2,5-diméthoxyphényl)-N-propylamino]thiazole
. 4-(2-Chloro-4-methoxyphényl)-5-methyl-2-[N-(2-méthoxy-5-méthoxycarbonylphényl)-N-propylamino]thiazole
. 4-(2,4-Dichlorophényl)-5-méthyl-2-[N-(2,5-dichlorophényl)-N-propylamino]thiazole,
. 4-(2-Chloro-4-méthoxyphényl)-5-méthyl-2-[N-(5-acétyl-2-méthoxyphényl)-N-propylamino]thiazole
. 4-(2-Chloro-4-méthoxyphényl)-5-méthyl-2-[N-(2-(méthoxy)-5-(phényl)phényl)-N-propylamino]thiazole
. 4-(2-Chloro-4-méthoxyphényl)-5-méthyl-2-[N-(2,6-diméthoxyphényl)-N-propylamino]thiazole
. 4-(2-Chloro-4-méthoxyphényl)-5-méthyl-2-[N-(2-méthoxy-6-méthylphényl)-N-propylamino]thiazole
. 4-(2,4-dichlorophényl)-5-méthyl-2-[N-(2-méthoxy-6-méthylphényl)-N-propylamino]thiazole
. 4-(2-Chloro-4-méthoxyphényl)-5-méthyl-2-[N-(5-éthyl-2-méthoxyphényl)-N-propylamino]thiazole
. 4-(2-Chloro-4-méthoxyphényl)-5-méthyl-2-[N-(5-bromo-2-méthoxyphényl)-N-propylamino]thiazole
. 4-(2-Chloro-4-méthoxyphényl)-5-méthyl-2-[N-(2-méthoxy-5-o-tolylphényl)-N-propylamino]thiazole
. 4-(2,4,6-Trichlorophényl)-5-méthyl-2-[N-(2,5-ditrifluorométhylphényl)-N-propylamino]thiazole
. 4-(2,4-Dichlorophényl)-5-méthyl-2-[N-(2,5-ditrifluorométhylphényl)-N-propylamino]thiazole
. 4-(2-Chloro-4-méthoxyphényl)-5-méthyl-2-[N-(2-méthoxy-5-nitrophényl)-N-propylamino]thiazole
. 4-(2,4-Dichlorophényl)-5-méthyl-2-[N-(2,6-dichloro-5-méthylphényl)-N-propylamino]thiazole
. 4-(2,4-Dichlorophényl)-5-méthyl-2-[N-(2-méthoxy-6-méthylphényl)-N-propylamino]thiazole
. 4-(2,4-Dichlorophényl)-5-méthyl-2-[N-(5-chloro-2-méthylphényl)-N-propylamino]thiazole
. 4-(4-Chloro-2-trifluorométhylphényl)-5-méthyl-2-[N-(5-chloro-2-méthylphényl)-N-propylamino]thiazole
. 4-(2-Chloro-4-méthoxy-5-méthylphényl)-5-méthyl-2-[N-(2-méthoxy-5-méthylphényl)-N-propylamino]thiazole
. 4-(2-Chloro-4-méthoxyphényl)-5-méthyl-2-[N-(2-méthylthio-5-trifluorométhylphényl)-N-propylamino]thiazole
. 4-(2,4-Dichloro-5-méthylphényl)-5-méthyl-2-[N-(-2-méthoxy-5-méthylphényl)-N-propylamino]thiazole
. 4-(2-Chloro-4,5-diméthylphényl)-5-méthyl-2-[N-(2-méthoxy-5-méthylphényl)-N-propylamino]thiazole
. 4-(2-Chloro-4-méthoxyphényl)-5-méthyl-2-[N-(2-méthoxy-5-méthoxycarbonylphényl) -N-propylamino]thiazole
. 4-(2-Chloro-4-méthoxyphényl)-5-méthyl-2-[N-(5-chloro-2-méthylphényl)-N-prop-2-ynylamino]thiazole
un de leurs stéréoisomères, un de leurs sels, un de leurs hydrates et/ou un de leurs solvates.

Les composés de l'invention sous forme libre présentent, généralement des propriétés basiques. Toutefois suivant la nature des substituants certains peuvent présenter des propnétés acides.

Les sels des composés de formule (I) avec des acides ou des bases (lorsque cela est possible) pharmaceutiquement acceptables sont les sels préférés, mais ceux qui peuvent permettre d'isoler les composés de formule (I) notamment de les purifier ou d'obtenir des isomères purs, sont aussi objet de l'invention.

Parmi les acides pharmaceutiquement acceptables pour la préparation des sels d'addition aux composés de formule(I), on peut citer les acides chlorhydrique, phosphorique, fumarique, citrique, oxalique, sulfurique, ascorbique, tartrique, maléique, mandélique, méthanesulfonique, lactobionique, gluconique, glucarique, succinique, sulfonique, hydroxypropane sulfonique.

Parmi les bases pharmaceutiquement acceptables pour la préparation des sels d'addition aux composés de formule (I) lorsque ceux-ci ont des propriétés acides. on peut citer l'hydroxyde de sodium, de potassium ou d'ammonium.

Les composes selon l'invention ainsi que les intermédiaires utiles pour leur préparation sont préparés selon des méthodes bien connues de l'homme de l'art, en particulier selon EP 576 350 et EP 659 747.

Le schéma réactionnel suivant illustre un procédé de préparation pour la synthèse des composés (I).

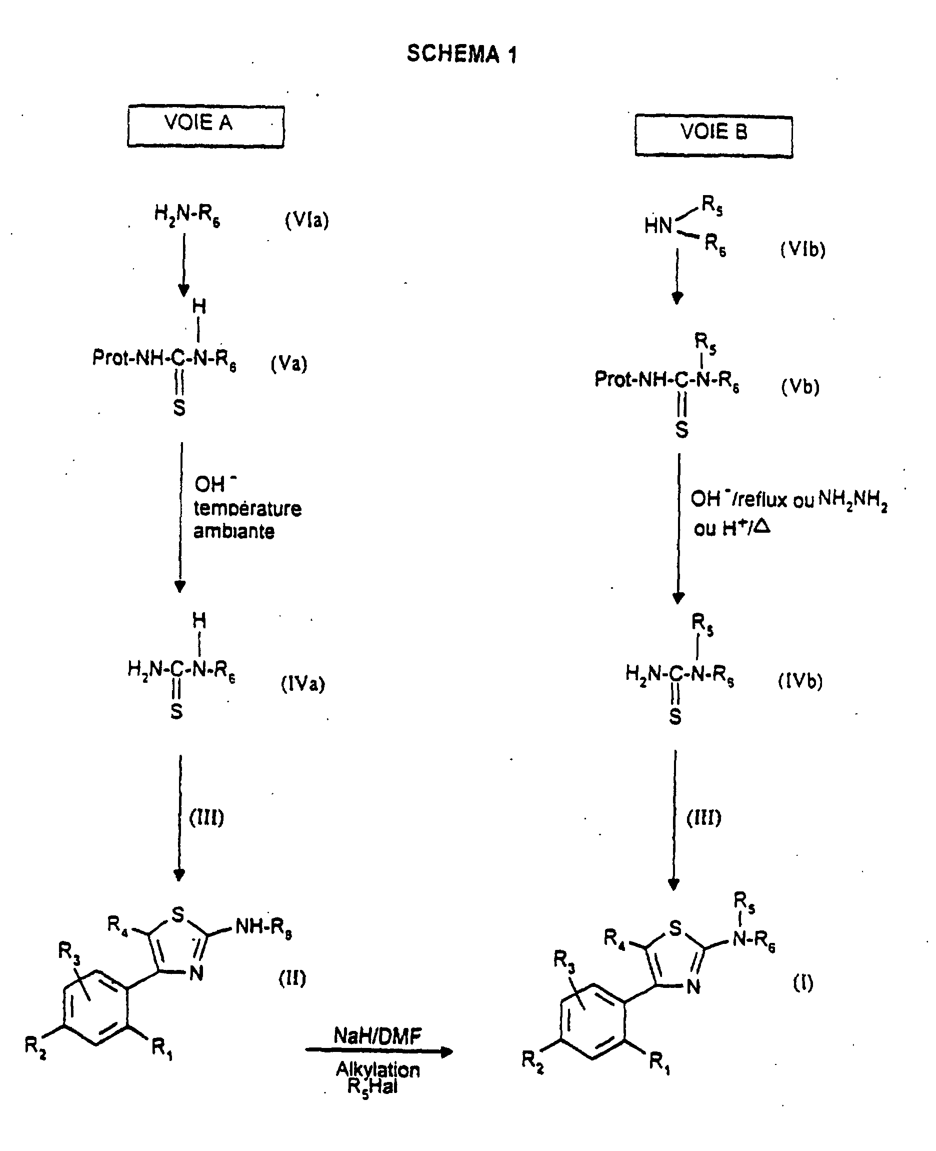

Selon un autre de ses aspects, la présente invention a également pour objet un procédé pour la préparation des composés de formule (I) caractérisé en ce que l'on fait réagir un dérivé alpha-halogéné, de préférence alpha-bromé ou alpha-chloré de formule (III) 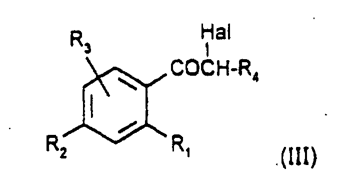 dans laquelle R₁, R₂, R₃ et R₄ sont tels que définis pour (I) et Hal représente un atome d'halogène de préférence de brome ou de chlore
soit avec une thiourée (VOIE A) de formule : 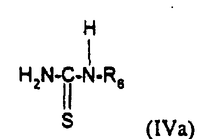 dans laquelle R₆ est tel que défini pour (I) pour obtenir un composé de formule (II) 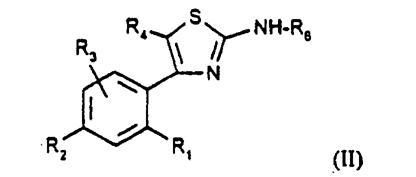 dans laquelle R₁, R₂, R₃, R₄ et R₆ sont tels que définis pour (I) pour le soumettre ensuite à une réaction d'alkylation pour fournir le composé (I),
soit avec une thiourée (VOIE B) de formule 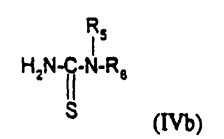 dans laquelle R₅ et R₆ sont tels que définis pour (I) pour conduire directement au composé (I),
et le cas échéant, les composés de formule (I) ainsi obtenus sont ensuite éventuellement séparés en leurs stéréoisomères possibles et/ou salifiés pour former les sels correspondants.

Les réactions d'alkylation mises en oeuvre dans le procédé ci-dessus sont réalisées dans les conditions habituelles connues de l'homme de l'art par action d'un agent alkylant approprié tel que, par exemple, un halogénure d'alkyle en présence d'une base de préférence l'hydrure de sodium.

Les dérivés de formule (III) peuvent être obtenus à partir des cétones correspondantes non halogénées de formule 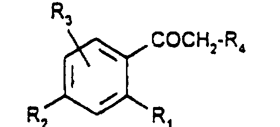 dans laquelle R₁, R₂, R₃ et R₄ sont tels que définis pour (I) soit (i) par action du brome dans un solvant organique approprié, tel que l'acide acétique, le tétrachlorure de carbone ou l'éther diéthylique, soit (ii) par action des tribromures d'ammonium quaternaires selon la méthode décrite dans Bull. Chem. Soc. Japan, 1987, *60*, 1159-1160 et 2667-2668. soit (iii) encore par action du bromure cuivrique dans un solvant organique, tel qu'un mélange de chloroforme et d'acétate d'éthyle selon J. Org. Chem. 1964, 29, 3451-3461. En variante, les composés de formule (III) peuvent êre obtenus par action du bromure de 2-bromopropionyle sur un benzène substitue de formule 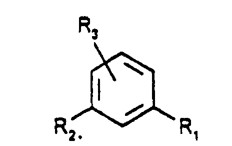 par une réaction de Friedel et Crafts.

Les cétones mentionnées ci-dessus sont en général des produits connus ou disponibles dans le commerce. Ces composés peuvent être préparés par réaction de Friedel et Crafts, en présence d'un acide de Lewis selon des méthodes bien connues de l'homme de l'art.

Les dérivés de thiourées (IVa) et (IVb) sont obtenus à partir des composés 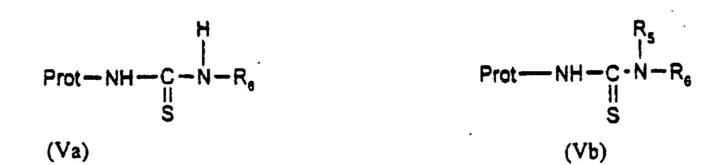 dans lesquels Prot représente un groupe protecteur, par exemple benzoyle ou pivaloyle, R₅ et R₆ étant tels que définis précédemment pour (I) soit par un traitement basique, en utilisant de préférence l'ammoniaque, l'hydroxyde de sodium ou de l'hydrazine à une température allant de la température ambiante au reflux du mélange réactionnel, soit par un traitement acide en utilisant de préférence l'acide chlorhydrique.

Les composés de formule (Va) et (Vb) sont préparés en faisant réagir selon des méthodes connues. un isothiocyanate, par exemple un isothiocyanate de benzoyle ou un isothsocyanate de pivaloyle sur les amines correspondantes de formule (VIa) et (VIb) 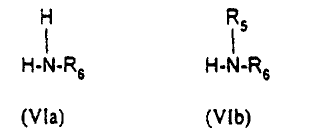 dans lesquelles R₅ et R₆ sont tels que définis pour (I).

Les amines secondaires (VIb) sont préparées à partir des amines primaires

R₆-NH₂ (VIa)

. soit par réaction avec un aldéhyde de formule R'₅-CHO dans lequel R'₅ représente R₅ tel que défini pour (I) diminué d'un atome de carbone dans la partie alkyle linéaire, puis réduction de l'imine par un hydrure alcalin, par exemple par NaBH₄ dans un alcanol de préférence dans l'éthanol ou le méthanol à température ambiante, ou par réaction avec un acide de formule R'₅COOH qui sert à la fois de réactif et de solvant, l'aldéhyde étant formé *in situ* lors de l'addition de l'hydrure alcalin,
. soit par réaction avec un halogénure d'acide ou un anhydride d'acide dans un solvant organique choisi parmi les hydrocarbures halogénés, tel que le dichlorométhane, en présence d'un accepteur de protons, de préférence la triéthylamine. L'amide issu de cette réaction est ensuite réduit par un hydrure alcalin tel que AlLiH₄ dans des solvants organiques de type éther diéthylique.

Les composés de formule (I), ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans les travaux de recherche, de métabolisme ou de pharmacocinétique, ou encore dans des essais biochimiques en tant que ligands de récepteur.

Les composés de la présente invention ont fait l'objet d'études biochimiques et pharmacologiques. Ils possèdent des propriétés pharmacologiques fort intéressantes. Les composés de l'invention déplacent, à des concentrations inférieures à 10 µM, la liaison du CRF ou de peptides apparentés iodés (urotensine, sauvagine) aux récepteurs spécifiques présents sur membranes de cerveaux animaux (rat, souris) ou humains et sur cellules en culture, selon la méthode décrite par E.B. De Souza (J. Neurosci., 1987, *7*, 1, 88-100).

Cela est étonnant et inattendu, puisque des composés de structure proche de celle des composés de l'invention ne déplacent pas de manière significative cette liaison.

Le CRF est un neuropeptide qui contrôle l'activité de l'axe hypothalamo-hypophyso-surrénalien. Ce facteur est responsable des réponses endocnnes et comportementales liées au stress.

En effet, il a été démontré que le CRF peut moduler le comportement comme aussi certaines fonctions du système nerveux autonome (G.F. Koob, F.E. Bloom, Fed. Proc., 1985, *44*, 259 ; M.R. Brown, L.A. Fisher, Fed. Proc., 1985, *44*, 243). Plus particulièrement, le CRF induit la sécrétion de la corticotropine (ACTH), des β-endorphines et autres peptides dérivés de la pro-opiomélanocortine (A. Tazi *et al*., Régul. Peptides, 1987, *18*, 37 ; M.R. Brown *et al*, Regul. Peptides, 1986, *16*, 321 ; C.L. Williams *et al*., Am. J. Physiol., 1987, G 582, 253).

Les composés de l'invention peuvent être donc utiles à la régulation de la sécrétion de ces substances endogènes. Ils trouvent plus spécialement leurs applications en tant que principes actifs des médicaments pour diminuer la réponse au stress (comportement, états émotionnels, troubles gastro-intestinaux et cardiovasculaires, désordres du système immunitaire) et plus généralement dans les pathologies impliquant le CRF, par exemple les désordres psychiatriques, l'anxiété, la dépression, l'anorexie nerveuse, les troubles de l'activité sexuelle et de la fertilité, la maladie d'Alzheimer ou autres.

Les composés de l'invention sont très stables et sont donc ainsi particulièrement appropriés pour constituer le principe actif de médicaments.

L'invention s'étend aussi aux compositions pharmaceutiques contenant comme principe actif, un composé de formule (I) ou l'un de ses sels pharmaceutiquement acceptables, éventuellement en association avec un ou plusieurs excipients inertes et appropriés.

Dans chaque unité de dosage le principe actif de formule (I) est présent dans les quantités adaptées aux doses journalières envisagées. Chaque unité de dosage est convenablement ajustée selon le dosage et le type d' administration prévu, par exemple comprimés, gélules et similaires, sachets, ampoules, sirops et similaires, gouttes, patch transdermique ou transmucosal de façon à ce qu'une telle unité de dosage contienne 0,5 à 800 mg de principe actif, de préférence 0,5 à 200 mg devant être administrés chaque jour.

Les composés selon l'invention peuvent également être utilisés en association avec un autre principe actif utile pour la thérapeutique souhaitée tels que par exemple des anxiolytiques, des antidépresseurs ou des anorexigènes.

Les composés de formule (1) sont peu toxiques leur toxicité est compatible avec leur utilisation comme médicament pour le traitement des troubles et des maladies ci-dessus.

Les composés de formule (I), peuvent être formulés dans des compositions pharmaceutiques pour l'administration aux mammifères, y compris l'homme, pour le traitement des maladies susdites.

Les compositions pharmaceutiques ainsi obtenues sont présentées avantageusement sous des formes diverses, telles que par exemple, des solutions injectables ou buvables, dragées, comprimés ou gélules. Les compositions pharmaceutiques contenant comme principe actif au moins un composé de formule (I) ou l'un de ses sels, sont notamment utiles pour le traitement à titre préventif ou curatif, des maladies liées au stress et plus généralement dans le traitement de toutes les pathologies impliquant le CRF, tels que par exemple : la maladie de Cushing, les désordres neuropsychiatriques comme la dépression, l'anxiété, la panique, les désordres compulsifs obsessionnels, les troubles de l'humeur, les troubles du comportement, l'agressivité, l'anorexie, la boulimie, l'hyperglycémie, la croissance retardée, les troubles du sommeil, l'épilepsie et les dépressions de tout types ; la maladie d'Alzheimer, de Parkinson ; la chorée de Huntington ; la sclérose latérale amyotrophique ; les troubles vasculaires, cardiaques et cérébraux; les troubles de l'activité sexuelle et de la fertilité ; l'immunodépression, l'immunosuppression, les processus inflammatoires, les infections multiples, l'arthrite rhumatoïde, l'osteo-arthrite, les uvéites, le psoriasis ainsi que le diabète; les cancers; les troubles gastro-intestinaux et les inflammations qui en découlent (colon irritable et inflammatoire, diarrhées) ; les troubles de perception de la douleur, les fibromyalgies liées ou non aux troubles du sommeil, la fatigue, la migraine ; les symptômes liés à la dépendance (alcoolique) et au sevrage de drogues.

La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,5 mg à 800 mg par jour, de préférence d'environ 0,5 à 200 mg par jour.

Dans les compositions pharmaceutiques de la présente invention pour l'administration orale, sublinguale, sous-cutanée, intramusculaire, intraveineuse, transdermique, transmucosale, locale ou rectale, le principe actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispérsion et/ou des agents mouillants pharmacologiquement compatibles, par exemple le propylèneglycol ou le butylèneglycol.

Pour une administration transmucosale le principe actif peut être formulé en présence d'un promoteur tel qu'un sel biliaire, d'un polymère hydrophile tel que par exemple l'hydroxypropylcellulose, l'hydroxypropylméthylcellulose, l'hydroxyéthylcellulose, l'éthylcellulose, la carboxyméthylcellulose, le dextran, la polyvinylpyrrolidone, les pectines, les amidons, la gelatine, la caséine, les acides acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Le principe actif peut être formulé également sous forme de microcapsules, éventuellement avec un ou plusieurs supports ou additifs.

Le principe actif peut être également présenté sous forme de complexe avec une cyclodextrine, par exemple α-, β- ou γ- cyclodextrine, 2-hydroxypropyl-β-cyclodextrine ou méthyl-β-cyclodextrine.

Les EXEMPLES suivants, donnés à titre non limitatif, illustrent l'invention.

Dans les PREPARATIONS sont décrites les méthodes de synthèse des différents intermédiaires permettant d'obtenir les composés de l'invention. Ces intermédiaires sont tous obtenus selon des méthodes bien connues de l'homme de l'art.

Les points de fusion ont été mesurés selon la technique Micro-Köfler et sont exprimés en degré Celsius.

Les spectres de résonance magnétique nucléaire du proton (¹H RMN) des composés de formule (I) ont été enregistrés, selon le cas, à 200 MHz ou à 100 MHz. Les déplacements chimiques sont donnés en ppm et les constantes de couplage en Hertz.

Les composés de l'invention présentent une analyse centésimale conforme à la théorie.

Les composés de l'invention décrits dans le TABLEAU I ont également des spectres RMN conformes à leur structure.

### PREPARATIONS

### PREPARATION DES CETONES DE FORMULE III

### PREPARATION I

### 2-Bromo-1-(2,4-dichlorophényl)propan-1-one (Composé 1-1)

A 7 g de 1-(2,4-dichlorophényl)propan-1-one en solution dans un mélange de 420 ml de dichlorométhane et de 140 ml de méthanol, on ajoute 17,4 g de tribromure de tetrabutylammonium à température ambiante. Après 24 heures le mélange réactionnel est concentré sous vide. Le résidu est repris à l'eau, extrait avec de l'acétate d'éthyle, on sèche la phase organique avec du sulfate de sodium et évapore sous vide. puis purifie sur colonne de gel de silice avec comme éluant un mélange de cyclohexane/acétate d'éthyle 20/1 (v/v) pour obtenir une huile.

De la même manière peut aussi être obtenu en utilisant les cétones adéquates le composé suivant :
2-bromo-1-(2-chloro-4-méthoxyphényl)propan-1-one *(Composé 1-2)*

### PREPARATION II

### 2-Bromo-1-(2-chloro-4-trifluorométhylphényl)propan-1-one (Composé 2-1)

**Etape 1 :** A 0°C, sous atmosphère inerte, 63 ml d'une solution (3M) d'éthyl magnésium dans l'éther diéthylique est ajoutée à une solution de 19,5 g de 3-chloro-4-cyanobenzotrifluorure dans 250 ml d'éther. Après 4 heures de réaction à température ambiante le mélange réactionnel est à nouveau refroidi à 0°C. On ajoute 5 ml d'acide chlorhydrique (10 N). L'addition terminée, le mélange est chauffé au reflux (60°C) durant 1 heure. De retour à température ambiante, on extrait la phase aqueuse à l'éther puis au dichlorométhane. Les phases organiques sont lavées avec une solution saturée en NaCl avant d'être séchées sur sulfate de sodium. Après évaporation sous vide, le résidu est purifié sur gel de silice, éluant cyclohexane/acétate d'éthyle 20/1 (v/v) ; R = 92 %.
RMN ¹H (DMSO) : 1,10(*t*,3H) ; 2,94(*q*,2H) ; 7,84(*s*,2H) ; 7,92(*s*,1H),

**Etape 2 :** A température ambiante, à 11,8 g du produit obtenu précédemment en solution dans 500 ml de dichlorométhane on ajoute 26 g de tribromure de tétrabutylammonium. Le mélange est alors chauffé pendant 3 heures à 35-40°C. De nouveau à température ambiante, on lave la phase organique à l'eau puis avec une solution saturée en NaCl avant de la sécher sur sulfate de sodium. On évapore sous vide ; R = 97 %.
RMN ¹H (CDCl₃) : 0.89(*d*,3H) ; 5,16(*q*,1H) ; 7,61(*m*,2H) ; 7,67(*m*,1H).

### PREPARATION III

### 2-Bromo-1-(2,4,6-trichlorophényl)éthan-1-one (Composé 3-1)

A 0°C, sous argon, à une solution de 31,1 g de 1,3,5-trichlorobenzène. on additionne 15,2 ml de bromure de bromoacétyle puis de 23,3 g de chlorure d'aluminium par petites portions (en 2 heures). Le mélange réactionnel est alors chauffé pendant 8 heures à 80°C. De retour à température ambiante on dilue au dichlorométhane puis on additionne, à 0°C, de l'acide chlorhydrique (1N). La phase aqueuse est extraite au dichlorométhane, les phases organiques sont rassemblées, lavées à l'eau puis séchées sur sulfate de sodium. Le résidu d'évaporation est purifié sur silice ; R = 70 %.
RMN ¹H(DMSO) : 4,77(*s*,2H) ; 7,85(*s*,2H).

Une variante de ce procédé consiste à utiliser un solvant CH₂Cl₂ et à procéder à une température voisine de 10°C en partant du 1,3-dichloro-4-méthylbenzène on obtient la 2-bromo-1-(2,4-dichloro-5-méthylphényl)éthan-1-one *(Composé 3-2).*

On prépare de la même façon la 2-bromo-1-(2-chloro-4,5-diméthylphényl)éthan-1-one *(Composé 3-3).*

### PREPARATION DES AMINES

### PREPARATION IV

### 6-Amino-7-méthoxy-1,2,3,4-tétrahydronaphtalène (Composé 4-1)

**Etape 1 :** Dans 100 ml d'anhydride acétique, on dissout 12,0 ml de 7-méthoxy-1,2,3,4-tétrahydronaphtaléne puis on ajoute 3,6 ml d'acide nitrique fumant dissout dans 10 ml d'anhydride acétique. On agite le mélange réactionnel pendant une heure à température ambiante.

Après avoir ajouter de la glace, on neutralise avec de l'hydroxyde de sodium 10N et on extrait à l'acétate d'éthyle. On sèche sur sulfate de sodium puis on évapore à sec. Le résidu obtenu est purifié par chromatographie sur colonne de gel de silice éluée avec un mélange acétate d'éthyle/hexane 10/90 (v/v) pour la séparation des 2 isomères, 3,3 g de produit attendu sont obtenus sous forme de cristaux jaunes.

**Etape 2** : On dissout 3,2 g du produit obtenu précédemment dans 60 ml d'acide acétique et 30 ml d'acide chlorhydrique concentré puis on ajoute 10 g de SnCl₂, H₂O et on chauffe le mélange réactionnel à reflux pendant 3 heures. On évapore puis on reprend le résidu obtenu par une solution de carbonate de sodium saturée et on extrait à l'acétate d'éthyle. On sèche sur sulfate de sodium puis on évapore à sec. 2,65 g de produit attendu sont obtenus sous forme de cristaux ; R = 97 %.
¹H RMN(CDCl₃ . 1,74-1,80(*m*,4H, -C*H*₂-C*H*₂-) ; 2,64-2,68(*m*,4H, -C*H*₂-CH₂-CH₂-C*H*₂) : 3,67(*m*, 2H, -N*H*₂) ; 3,68(*s*,3H, -OC*H*_{*3*}) ; 6,46(*s*,1H, *H*_{*1*}) ; 6,51(*s*,1H,*H*_{*4*}).

### PREPARATION V

### N-Propyl-2,4-dichloroaniline (Composé 5-1)

**Etape 1 :** A une solution de 9,2 g de 2,4-dichloroaniline dans 100 ml de chlorure de méthyléne anhydre, on ajoute sous argon et à 0°C 9,5 ml de triéthylamine puis on ajoute goute à goutte 5,9 ml de chlorure de propionyle. On laisse ensuite remonter la température du mélange réactionnel jusqu'à température ambiante. On lave la phase organique à l'eau puis avec une solution saturée de NaCl, on sèche sur sulfate de sodium et on évapore à sec sous vide. Après solubilisation du résidu obtenu dans du dichlorométhane à chaud, on ajoute de l'éther isopropylique.
9,6 g du produit désiré sont obtenus sous forme de cristaux blancs ; R = 80 % ; F = 121°C.

**Etape 2 :** A 9,6 g du produit précédemment obtenu en solution dans 80 ml de tétrahydrofurane, on ajoute lentement et sous argon 50 ml d'une solution d'hydrure de lithium-aluminium 1 M dans du tétrahydrofurane. On chauffe le mélange réactionnel à reflux pendant 2 heures. A 0°C, on détruit l'excès d'hydrure de lithium-aluminium avec précaution par addition d'eau puis d'hydroxyde de sodium. Après élimination du précipité, la solution organique est évaporée à sec sous vide. On reprend le résidu obtenu par de l'éther diéthylique puis on extrait par une solution d'acide chlorhydrique 1N. On amène la phase aqueuse à pH=12 et on extrait par du dichlorométhane. On sèche la phase organique sur du sulfate de sodium puis on évapore. 7,5 g du produit attendu sont obtenus sous forme d'huile ; R = 84 %. ¹H RMN (CDCl3) : 0,99(*t*,2H) ; 1,57(*m*,2H) ; 3,01(*t*,2H) ; 4,27(1H) ; 6,4-7,2(*m*,3H).

En procédant comme indiqué pour la PREPARATION V, ci-dessus, et en utilisant comme matières premières les amines primaires adéquates, on prépare :
. la N-Propyl-2-chloroaniline *(Composé 5-2)*
. la N-Propyl-3-chloroaniline *(Composé 5-3)*
. la N-Propyl-2-méthylaniline *(Composé 5-4)*
. la N-Propyl-2-méthoxyaniline *(Composé 5-5)*
. la N-Propyl-2-trifluorométhyloxyaniline *(Composé 5-6)*
. la N-Propyl-2,6-dichloroaniline *(Composé 5-7)*
. la N-Propyl-2,5-dichloroaniline *(Composé 5-8)*
. la N-Propyl-2-chloro-5-trifluorométhylaniline *(Composé 5-9)*
. la N-Propyl-2-chloro-5-méthoxyaniline *(Composé 5-10)*
. la N-Propyl-5-chloro-2-méthoxyaniline *(Composé 5-11)*
. la N-Propyl-5-chloro-2-éthoxyaniline (*Composé 5-12)*
. la N-Propyl-2,5-diméthylaniline *(Composé 5-13)*
. la N-Propyl-2-méthoxy-5-méthylaniline *(Composé 5-14)*
. la N-Propyl-2-méthoxy-5-trifluorométhylaniline *(Composé 5-15)*
. la N-Propyl-2,5-diméthoxyaniline (*Composé 5-16)*
. la N-Propyl-2,4.6-trichioroaniline *(Composé 5-17)*
. la N-Propyl-2,6-diméthoxyaniline *(Composé 5-18)*

### PREPARATION VI

### N-Propyl-5-bromo-2-méthoxyaniline (Composé 6-1)

**Etape 1 :** en procédant selon Simada T. (Sci. Pap. Inst. Phys. Chem. Rés. Jpn., vol. 35, n° 884, pp 365-371, 1938). le 5-bromo-2-méthoxynitrobenzène est obtenu à partir du 4-bromo-2-nitro phénol.
RMN ¹H (CDCl₃) ; 3,94(*s*,3H) ; 7,06(*d*,1H) ; 7,64(*dd*,1H) ; 8,23(*d*,1H).

**Etape 2 :** le produit obtenu précédemment est réduit en présence de fer et d'acide chlorhyanque concentré dans un mélange éthanol, eau. Le 5-bromo-2-méthoxyaniline obtenu subit une acylation suivie d'une réduction tel que décrit dans la PREPARATION V.
RMN ¹H(CDCl₃) : 1,00(*t*,3H) ; 1,66(*m*,2H) ; 3,05(*t*,2H) ; 3,81(*s*,3H) ; 4,25(*m*,1H) ; 6,55-6.74(*m*,3H).

### PREPARATION VII

### N-Propyl-2-méthoxy-6-méthylaniline (Composé 7-1)

**Etape 1 :** à partir du 2-méthoxy-6-méthylaniline on obtient le N-propionyl-2-méthoxy-6-méthylaniline en suivant le protocole décrit précédemment (PREPARATION V, Etape 1).
RMN ¹H (CDCl₃) : 1,29(*t*,3H) ; 2,21(*s*,3H) ; 2,48(*q*,2H) ; 3,78(*s*,3H) ; 6,70-6,83(*m*,3H); 7,06-7,14(*m*,1H).

**Etape 2 :** à 0°C, sous argon, 11,9 g de borohydrure de sodium (3,5 équivalents) sont ajoutés à 180 ml d'une solution 1 M dans le dichlorométhane de tétrachlorure de titane (2 équivalents). La solution de 19 g de N-propionyl-2-méthoxy-6-méthylaniline dans 100 ml de diméthoxyéthane est ajoutée lentement au mélange réactionnel de façon à ce que la température du mélange se situe entre 5 et 15°C. L'addition terminée, le mélange est chauffé aux alentours de 65°C. A 0°C, le mélange est alors hydrolysé trés lentement puis alcalinisé à l'aide d'hydroxyde de sodium. La phase aqueuse est extraite au dichlorométhane.

Les phases organiques sont lavées à l'eau puis avec une solution saturée en NaCl, avant d'être séchées sur sulfate de sodium et évaporées sous vide ; R = 58 %.
RMN ¹H (CDCl₃) : 0,95(*t*,3H) ; 1,57(*m*,2H) ; 2,27(*s*,3H) ; 3,00(*t*,2H) ; 3,77(*s*,3H) ; 6,65-6,80(*m*,3H).

### PREPARATION VIII

### N-Propyl-2-méthoxy-5-méthoxycarbonylaniline (Composé 8-1)

On met en solution 15 g de 2-méthoxy-5-méthoxycarbonylaniline dans 500 ml d'acide propionique sous argon et à température ambiante et on ajoute par petites portions 15,7 g de borohydrure de sodium. On agite le mélange réactionnel pendant 2 heures puis on hydrolyse et on alcalinise par de l'hydroxyde de sodium 10N en maintenant la température au dessous de 20°C. On extrait au dichlorométhane, on lave la phase organique avec une solution saturée de NaCl, on séche puis on évapore sous vide. Le produit attendu est obtenu sous forme d'huile avec un rendement quantitatif.

En procédant comme indiqué pour la PREPARATION VIII, ci-dessus, et en utilisant l'amine primaire adéquate, on prépare la N-propyl-2,5-ditrifluorométhylaniline *(Composé 8-2).*
. N-Propyl-2-chloro-5-méthylaniline *(Composé 8-3)*
. N-Propyl-2-méthoxy-5-phénylaniline *(Composé 8-4)*
. N-Propyl-5-éthyl-2-méthoxyaniline (*Composé 8-5)*
. N-Propyl-5-chloro-2,4-diméthoxyaniline *(Composé 8-6)*
. N-Propyl-4-chloro-2,5-diméthoxyaniline *(Composé 8-7)*
. N-Propyl-2-chloro-5-méthoxy-4-méthoxycarbonylaniline *(Composé 8-8)*
. N-Propyl-2-thiométhyl-5-trifluorométhylaniline *(Composé 8-9)*
. N-Propyl-2-méthyl-5-méthoxycarbonylaniline *(Composé 8-10)*

### PREPARATION DES THIOUREES

### PREPARATION IX

### N-(7-Méthoxy-1,2,3,4-tétrahydronapht-6-yl)thiourée (Composé 9-1)

On dissout 1,45 g de thiocyanate d'ammonium dans 30 ml d'acétone et après avoir ajouté 2.2 ml de chlorure de benzoyle, on chauffe le mélange réactionnel à reflux pendant 15 minutes. On ajoute 2,6 g de 6-amino-7-méthoxy-1,2,3,4-tétrahydronaphtalène *(Composé 4-1)* dissout dans 20 ml d'acétone, puis on chauffe à reflux pendant 30 minutes. On évapore à sec, on reprend le résidu obtenu dans un minimum d'éthanol, on ajoute 50 ml d'hydroxyde d'ammonium à 30 % et on chauffe à reflux pendant 20 heures. La thiourée obtenue est refroidie, filtrée, lavée à l'eau puis avec un mélange acétate d'éthyle/hexane 25/75 (v/v). 2,8 g de produit attendu sont obtenus sous forme de poudre beige ; R = 80%.

### PREPARATION X

### N-(2,4-Dichlorophényl)-N-propylthiourée (Composé 10-1)

**Etape 1 :** A 3,4 g de thiocyanate d'ammonium dans 80 ml d'acétone anhydre, on ajoute sous argon et à 0°C 5,1 ml de chlorure de benzoyle et on agite le mélange réactionnel pendant 15 minutes. On additionne 7,48 g de N-propyl-2,4-dichloroaniline *(Composé 5-1)* dans 60 ml d'acétone anhydre et on agite le mélange réactionnel pendant 2 heures à température ambiante. On évapore à sec, on reprend le résidu obtenu par du dichlorométhane, on lave à l'eau et on sèche. 13,5 g du produit attendu sont obtenus sous forme d'huile ; R = 82 %.

**Etape 2 :** A 13,5 g du produit précédemment obtenu en solution dans 150 ml de méthanol, on ajoute 3,6 ml d'hydrazine. On abandonne le mélange réactionnel peridant 12 heures à température ambiante. On évapore le méthanol, on reprend le résidu obtenu par du dichlorométhane puis on lave à l'eau et on évapore à sec. Le résidu est purifié par chromatographie sur colonne de gel de silice éluée par un mélange acétate d'éthylelcyclohexane 1/3 (v/v). 6,9 g de produit attendu sont obtenus sous forme de cristaux ; R = 71 % ; F = 121°C.

En procédant comme indiqué pour l'étape 1 de la PREPARATION X, ci-dessus, et en utilisant la N-propyl-2-méthoxy-5-méthoxycarbonylaniline *(Composé 8-1)*, on prépare la N'-benzoyl-N-(2-méthoxy-5-méthoxycarbonylphényl)-N-propylthiourée *(Composé 10-2).*

### PREPARATION XI

### N-(5-Chloro-2-méthylphényl)thiourée (Composé 11-1)

**Etape 1 :** On ajoute 8,9 ml de chlorure de benzoyle à une solution de 5,9 g de thiocyanate d'ammonium dans 130 ml d'acétone anhydre. On chauffe le mélange réactionnel au reflux pendant 15 minutes puis après avoir refroidi, on ajoute 10 g de 5-chloro-2-méthylaniline. On chauffe le mélange réactionnel à reflux pendant 2 heures et on élimine I 'acetone par distillation sous vide. On reprend le résidu obtenu dans du dichlorométhane. On lave à l'eau, on sèche sur sulfate de sodium et on évapore sous vide. Le produit attendu est obtenu sous forme d'huile avec un rendement quantitatif.

**Etape 2 :** Le produit obtenu précédemment est chauffé à 90°C pendant 16 heures en présence de 300 ml d'une solution d'hydroxyde de sodium à 5 %. Après avoir refroidi, on ajuste le mélange réactionnel à pH=7 par addition d'une solution d'acide chlorhydrique 1N. Le produit précipite et 10,53 g de produit attendu sont obtenus sous forme de cristaux jaunes ; R = 74 % ; F = 137°C.

### PREPARATION XII

### N-(5-Carboxy-2-méthoxyphényl)-N-propylthiourée (Composé 12-1)

Le produit attendu est obtenu à partir de la N'-benzoyl-N-(2-méthoxy-5-méthoxycarbonylphényl)-N-propylthiourée *(Composé 10-2)* en procédant selon la PREPARATION X et en utilisant un excès d'hydroxyde de sodium pour obtenir en plus de la déprotection de la thiourée, la saponification de l'ester.

En procédant selon les PREPARATIONS I à XII, ci dessus, on prépare en utilisant les produits de départ appropriés les intermédiaires permettant la synthèse des composés (I) selon l'invention.

### EXEMPLE 1

### Chlorhydrate de 4-(2-chloro-4-méthoxyphényl)-5-méthyl-2-[N-(7-méthoxy-1,2,3,4 -tétrahydronapht-6-yl)-N-propylamino]thiazole

(I) : R₁ = Cl ; R₂ = OCH₃ ; R₃ = H ; R₄ = CH₃ ; R₅ = -CH₂CH₂CH₃ R₆=

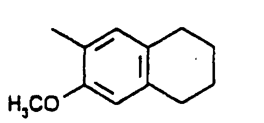

**Etape 1 :** On dissout 1,4 g de 2-bromo-1-(2-chloro-4-méthoxyphényl)propan-1-one *(Composé 1-2)* dans 50 ml de méthanol puis on ajoute 1,3 g de N-(7-méthoxy-1,2.3,4-tétrahydronapht-6-yl)thiourée *(Composé 9-1)*. On chauffe le mélange réactionnel à reflux pendant 12 heures, on évapore à sec puis on reprend le résidu obtenu dans une solution saturée de carbonate de sodium. On extrait à l'acétate d'éthyle, séche sur sulfate de sodium et on évapore à sec. Le résidu est purifié par chromatographie sur colonne de gel de silice éluée par un mélange acétate d'éthyle/hexane 25/75 (v/v). On reprend le précipité obtenu dans de l'éther de pétrole puis on le filtre.

1 3 g de produit attendu sont obtenus sous forme de poudre blanche (R = 62 %) H RMN(CDCl₃) 1,78-1.82(*m* 4H,-C*H*_{*2*}-C*H*_{*2*}). 2.24(*s*,3H,-C*H*_{*3*}), 2,73-2,75(*m*.4H-C*H*₂-C*H*₂-). 3,85(*s*,3H.OC*H*_{*3*}). 3.86(*s*,3H,-OC*H*_{*3*}) ; 6,58(*s*,1H, *H*_{*4*}) ; 6,87(*dd*,J=2,5,J=8.4 1H *H*_{*6*}). 7 02(*d*,J=2.5.1H *H*_{*2*}), 7,35(*d*,J=8.7,1H,*H*_{*6*}),7,46(*s*.1H, *H*_{*6*}) ; 7,46(*s*,1H*,H*_{*1*}) 7.47(*s*, 1H.-N*H*-).

**Etape 2 :** Dans une suspension de 0,17 g d'hydrure de sodium dans 20 ml de diméthylformamide on ajoute 1,0 g du produit précédemment obtenu dissout dans 20 ml de diméthylformamide anhydre. On ajoute 0,44 ml de bromopropane puis on agite le mélange réactionnel à température ambiante pendant une heure. On ajoute ensuite 100 ml d'une solution saturée de chlorure d'ammonium, extrait à l'acétate déthyle puis on lave la phase organique avec une solution de NaCl saturée.

On sèche sur du sulfate de sodium et on évapore à sec. Le résidu est purifié par chromatographie sur colonne de gel de silice éluée avec un mélange acétate d'éthyle/hexane 10/90 (v/v), 0,89 g de produit attendu est isolé sous forme d'huile incolore. Le chlorhydrate est obtenu en ajoutant dans le produit préalablement dissout dans de l'éther diéthylique, de l'éther de pétrole puis de l'éther diéthylique saturé en acide chlorhydrique gazeux jusqu'à précipitation totale du sel; F = 101°C.
¹H RMN(CDCl₃) : 0,85 (*t*, J=7,3, 3H, -CH₂-C*H*_{*3*}); 1,46-1,57 (*m*, 2H, -C*H*₂-CH_{*3*}) ; 1,72 (*m*, 4H, -C*H*_{*2*}-C*H*_{*2*}-); 1,99 (*s*, 3H, -C*H*_{*3*}) ; 2,65-2,77 (*m*, 4H, -C*H*_{*2*}-C*H*_{*2*}-); 3,73-3,83 (*m*, avec à 3,78 (s, 3H, -OC*H*_{*3*}) et à 3,83.(s, 3H, -OC*H*_{*3*}) ; 2H, -N-C*H*_{*2*}-C*H*_{*2*}); 6,96 (*s*, 1H, *H*_{*1*}); 7,04 (*dd,* J=2,5, J=8,4, 1H, *H*_{*5*}); 7,15 (*s*, 1H, *H*_{*4*}) ; 7,20 (*d*, J = 2.5, *H*_{*3*}) ; 7,43 (*d*, J=8,4, 1H, *H*_{*6*}).

### EXEMPLE 2

### 4-(2,4-Dichlorophenyl)-5-methyl-2[N-(2,4-dichlorophenyl)-N-propylamino] thiazole

(I) : R₁ = Cl ; R₂ = Cl ; R₃ = H ; R₄ = CH₃ ; R₅ = -CH₂CH₂CH₃ R₆ =

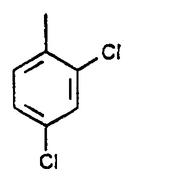

A 1,07 g de N-(2,4-Dichlorophényl)-N-propylthiourée *(Composé 10-1)* dans 6 ml d'éthanol, on ajoute 1 ml de triéthylamine puis on ajoute goutte à goutte 1,3 g de 2-bromo-1-(2,4-dichlorophényl)propan-1-one *(Composé 1-1).* On chauffe le mélange réactionnel pendant 3 heures à 78°C, on évapore à sec puis on reprend le résidu dans le dichlorométhane. On lave la phase organique à l'eau, on sèche sur sulfate de sodium et on évapore à sec sous vide.

Le résidu est purifié par chromatographie sur colonne de gel de silice éluée par un melange cyclohexane/acétate d'éthyle 3/1 (v/v). 1,31 g du produit attendu sont obtenus sous forme de cristaux dans du pentane : R = 72 % F = 90°C

### EXEMPLE 3

### 4-(2-Chloro-4-méthoxyphényl)-5-méthyl-2-[N-(5-carboxy-2-méthoxyphényl)-N-propylamino]thiazole

(I) : R₁ = Cl ; R₂ = OCH₃ ; R₃ = H ; R, = CH₃ ; R₅ = -CH₂CH₂CH₃ ; R₆ =

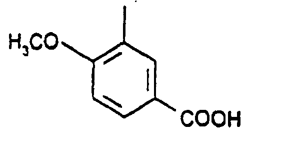

En suivant le procédé de l'EXEMPLE 2 et en utilisant le 2-bromo-1-(2-chloro-4-méthoxyphényl)propan-1-one, *(Composé 1-2*) et la N-(2-méthoxy-5-carboxyphényl)-N-propylthio-urée *(Composé 12-1)*, le produit attendu est obtenu sous forme de cristaux blancs ; R = 70% ; F = 109°C.

### EXEMPLE 4

### Chlorhydrate de 4-(2-chloro-4-méthoxyphényl)-5-méthyl-2-(N-(5-éthoxycarbonyl-2-méthoxyphényl)-N-propylamino]thiazole

(I) : R₁ = Cl ; R₂ = OCH₃ ; R₃ = H ; R₄ = CH₃ ; R₅ = -CH₂CH₂CH₃; R₆ =

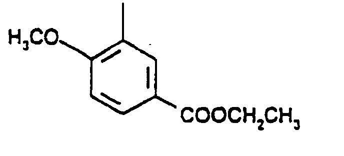

On met en solution 1 g de 4-(2-chloro-4-méthoxyphényl)-5-méthyl-2-[N-(5-carboxy-2-méthoxyphényl)-N-propylamino]thiazole (EXEMPLE 3) à température ambiante et sous argon dans 3 ml de diméthylformamide puis on ajoute 0,66 g de Cs₂CO₃ et 0,5 ml d'iodure d'éthyle. On agite pendant une heure le mélange réactionnel puis on le dilue par de l'acétate d'éthyle. On lave à l'eau, on sèche sur sulfate de sodium et on évapore à sec. Le résidu est purifié par chromatographie sur colonne de gel de silice éluée par un mélange cyclohexane/acétate d'éthyle 5/1 (v/v). Le produit attendu est obtenu sous forme d'une huile incolore.

On le reprend dans le dichloromethane et on ajoute une solution d'acide chlorhydrique 0,1N dans l'isopropanol. Après filtration, le produit attendu est obtenu sous forme de chlorhydrate. F = 111°C.

### EXEMPLE 5

### 4-(2-Chloro-4-méthoxyphényl)-5-méthyl)-2-(N-(2-méthoxy-5-morpholinocarbonyl-phényl)-N-propylamino]thiazole

(I) : R₁ = Cl ; R₂ = OCH₃ ; R₃ = H ; R₄ = CH₃ ; R₅ = -CH₂CH₂CH₃ ; R₆ =

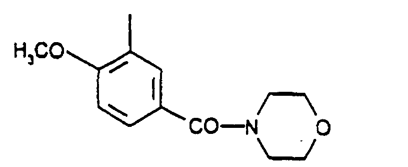

On ajoute 1 g de 4-(2-chloro-4-méthoxyphényl)-5-méthyl-2-[N-(5-carboxy-2-méthoxyphényl)-N-propylamino]thiazole (EXEMPLE 3) en solution dans 19 ml de diméthylformamide, puis on ajoute 0,31 ml de triéthylamine et 0,32 ml d'isobutylchloroformiate à -10°C et sous argon. On agite le mélange réactionnel pendant 10 minutes et on additionne 0,8 ml de morpholine. On laisse sous agitation pendant 2 heures à température ambiante. On dilue ensuite le mélange réactionnel dans l'acétate d'éthyle, on lave à l'eau, on sèche sur sulfate de sodium puis on évapore sous vide. Le résidu est purifié par chromatographie sur colonne de gel de silice éluée par un mélange cyclohexane/acétate d'éthyle 1/1 (v/v). Le produit attendu est obtenu sous forme d'une poudre ; F = 62°C.

### EXEMPLE 6

### 4-(2-Chloro-4-méthoxyphényl)-5-méthyl-2-[N-(5-acetyl-2-méthoxyphényl)-N-propylamino]thiazole

(I) : R₁ = Cl ; R₂ - OCH₃ ; R₃ = H ; R₄ = CH₃ ; R₅ = -CH₂CH₂CH₃ ; R₆ =

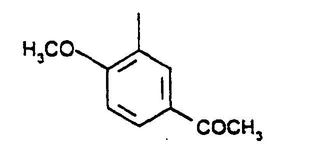

A 0°C, sous atmosphère inerte, 2 g de 4-(2-chloro-4-méthoxyphényl)-5-méthyl-2-[N-(5-carboxy-2-méthoxyphényl)-N-propylamino]thiazole sont mis en solution dans 30 ml d'éther diéthylique. Le méthyl lithium (3 équivalents) est ajouté lentement à la solution éthérée. L'addition terminée, le mélange reactionnel est amené à température ambiante. Après 2 heures, on dilue par de l'acétate d'ethyle puis on hydrolyse. La phase organique est lavée à l'eau, puis séchée sur sulfate de sodium. Après évaporation le résidu est purifié sur gel de silice. Le produit attendu est obtenu sous forme de poudre ; F = 50°C.

### EXEMPLE 7

### 4-(2-Chloro-4-méthoxyphényl)-5-méthyl-2-[N-(5-cyclopropylcarbonyl-2-méthoxyphényl)-N-propylamino]thiazole.

(I) : R₁ = Cl ; R₂ = OCH₃ ; R₃ = H ; R₄ = CH₃ ; R₅ = -CH₂H₂CH₃ ; R₆ =

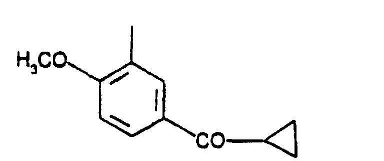

En partant du cyclopropane lithien et en utilisant le procédé de l'EXEMPLE 6, le produit attendu est obtenu sous forme de poudre ; F = 69°C.

### EXEMPLE 8

### Chlorhydrate de 4-(2-chloro-4-méthoxyphényl)-5-méthyl-2-[N-(5-isobutyryl-2-méthoxyphényl)-N-propylamino]thiazole.

(I) : R₁ = Cl ; R₂ = OCH₃ ; R₃ = H ; R₄ = CH₃ ; R₅ = -CH₂CH₂CH₃ ; R₆ =

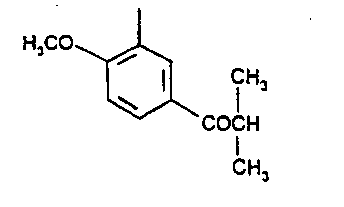

**Etape 1 :** sous atmosphère inerte, 6,5 g de 4-(2-chlore-4-méthoxyphényl)-5-méthyl-2-[N-(5-carboxy-2-méthoxyphényl)-N-propylamino]thiazole sont mis en solution dans 130 ml de diméthylformamide. La température du mélange est amenée à 0°C, 7,5 ml de triéthylamine sont additionnés ainsi que 2,8 ml de chloroformiate d'isobutyle (1,5 équivalents). Après 15 minutes, on ajoute 2,84 g de N-méthoxy-N-méthylamine (2 équivalents). La réaction est pratiquement achevée en 2 heures a temperature ambiante.

Le mélange réactionnel est alors dilué par de l'acétate d'éthyle. Après plusieurs lavages avec de l'eau puis avec une solution saturée en NaCl, la phase organique est séchée sur sulfate de sodium. Aprés évaporation le produit est purifié sur gel de silice, éluant dichlorométhane/acétate d'éthyle 95/5, (v/v) ; R = 72%.

RMN ¹H (CDCl₃) : 0,91(*t*,3H) ; 1,59(*m*,2H) ; 2,04(*s*,3H) ; 3,37(*s*,3H) ; 3,58(*s*,3H) ; 3,81 (*s*,3H) ; 3,85(*m*,2H) ; 3,88(*s*,3H) ; 6,81-6,87(*dd*,1H) ; 6,97-7,06(*m*,2H) ; 7,33-7,38(*d*,1H) ; 7,78-7,82(*m*,2H).

**Etape 2 :** la solution de 650 mg de produit obtenu précédemment sous atmosphère inerte dans 2 ml de tétrahydrofurane est additionnée lentement et à 0°C sur 1,4 ml d'une solution (2M), de chlorure d'isopropyle magnésien dans le tétrahydrofurane. Après un temps de réaction de 2 heures à température ambiante, le mélange réactionnel est à nouveau refroidi avant d'être acidifié par de l'acide chlorhydrique 1 N. La phase aqueuse est extraite à l'étherdiéthylique. Les phases organiques sont alors lavées à l'aide d'une solution saturée de bicarbonate de sodium puis à l'aide d'une solution saturée de chlorure de sodium.

Le résidu est séché sur sulfate de sodium, le solvant évaporé avant d'être purifié sur gel de silice, éluant cyclohexane (3), acétate d'éthyle (1). Le produit est obtenu sous forme de chlorhydrate F = 114°C.

### EXEMPLE 9

### 4-(2-Chloro-4-méthoxyphényl)-5-méthyl-2-[N-(5-hydroxyméthyl-2-méthoxyphényl)-N-propyl amino]thiazole.

(I) : R₁ = Cl ; R₂ = OCH₃ ; R₃ = H ; R₄ = CH₃ ; R₅ = -CH₂CH₂CH₃ ; R₆ =

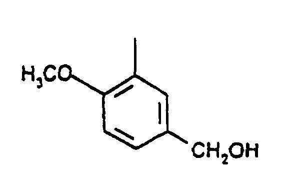

A 0°C. sous atmosphère inerte. 1,5 g de 4-(2-chloro-4-méthoxy phenyl)-5-méthyl-2-[N-(5-carboxy-2-méthoxyphenyl)-N-propylamino]thiazole sont mis en solution dans 11 ml de tétrahydrofurane On ajoute lentement de façon à ce que la température du mélange n'excède pas 10°C. 5 ml d'une solution 1M d'hydrure de lithium et d'aluminium dans le tétrahydrofurane. Après 2 heures à température ambiante, le mélange réactionnel est refroidi à l'aide d'un bain de glace puis hydrolysé lentement. Le brut est alcalinisé avant d'être filtré sur sulfate de sodium. Après évaporation sous vide, le produit est obtenu par purification sur gel de silice, éluant : dichlorométhanelacétate d'éthyle 95/5 (v/v) ; R = 87 %. Le produit attendu se présente sous forme de poudre ; F = 115°C.

### EXEMPLE 10

### Chlorhydrate de 4-(2-chloro-4-méthoxyphényl)-5-méthyl-2-[N-(2-méthoxy-5-méthoxyméthylphényl)-N-propylamino]thiazole.

(I) : R₁ = Cl ; R₂ = OCH₃ ; R₃ = H ; R₄ = CH₃ ; R₅ = -CH₂CH₂CH₃. R₆ =

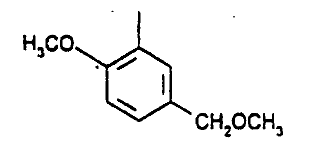

A 0°C, sous atmosphère inerte, 320 mg de 4-(2-chloro-4-méthoxyphényl)-5-méthyl-2-[N-(5-hydroxyméthyl-2-méthoxyphényl)-N-propylamino]thiazole sont mis en solution dans 0,7 ml de diméthylformamide. On ajoute 70 mg d'hydrure de sodium (50 % dans l'huile). Après 5 minutes, 0,24 ml d'iodure de méthyle sont additionnés. Après 12 heures à température ambiante, le mélange réactionnel est dilué avec de l'acétate d'éthyle puis hydrolysé. La phase organique est lavée plusieurs fois à l'eau avant d'être séchée sur sulfate de sodium. Après purification sur gel de silice, le produit attendu est mis en solution dans du dichlorométhane. Une solution d'acide chlorhydrique 0,1N dans l'isopropanol est alors ajouté. Après évaporation sous vide le produit attendu est obtenu sous forme de chlorhydrate ; F = 71°C.

### EXEMPLE 11

### Chlorhydrate de 4-(2-chloro-4-methoxyphényl)-5-méthyl-2-[N-(5-chloro-2-méthylphényl) -N-propylamino]thiazole

(I): R₁ = Cl ; R₂ = OCH₃ ; R₃ = H ; R₄ = CH₃ : R₅ = -CH₂CH₂CH₃ ; R₆ =

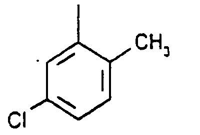

**Etape 1 :** A 3 g de N-(5-chloro-2-méthylphényl)thiourée *(Composé 11-1)* dans 25 ml d'éthanol, on ajoute 5 g de 2-bromo-1-(2-chloro-4-méthoxyphényl)propan-1-one *(Composé 1-2)* et 4,2 ml de triéthylamine dans 25 ml d'éthanol. On chauffe le mélange réactionnel à reflux pendant 2 heures et on réduit sous vide. On reprend le résidu ainsi obtenu dans du dichlorométhane. On lave à l'eau, on sèche sur sulfate de sodium et on évapore. 5,09 g de produit brut attendu sont obtenus. ¹H RMN (CDCl₃) : 2,14 (*s*, 3H) ; 2,23(*s*, 3H) ; 3,80 (*s*, 3H); 6,80 - 7,34 (*m*, 6H) ; 9,02 (*m*, 1H).

**Etape 2 :** On dissout 5,09 g du produit brut obtenu précédemment dans 70 ml de diméthylformamide anhydre et on ajoute 1,08 g d'hydrure de sodium sous argon. Après 15 minutes sous agitation, on additionne 4,4 g d'iodure de propyle et on chauffe le mélange réactionnel à 75 °C pendant 2 heures. On évapore, on hydrolyse le résidu obtenu et on extrait la phase aqueuse par de l'acétate d'éthyle. On lave la phase organique avec une solution saturée de NaCl, on sèche sur sulfate de sodium et on évapore sous vide. Le résidu est purifié par chromatographie sur colonne de gel de silice éluée par un mélange cyclohexane/acétate d'éthyle 20/1 (v/v).

On reprend le produit obtenu dans du dichlorométhane et on ajoute une solution d'acide chlorhydrique 0,1N dans l'isopropanol pour obtenir le chlorhydrate ; F = 69°C.

### EXEMPLE 12

### 4-(2-Chloro-4-méthoxyphényl)-5-méthyl-2-[N-(3,5-diméthylphényl)-N-propylamino]thiazole.

(I) : R₁ = Cl ; R₂ = OCH₃ ; R₃ = H ; R₄ = CH₃ ; R₅ = -CH₂CH₂CH₃ R₆ =

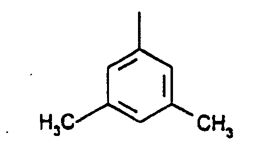

En procédant selon l'EXEMPLE 11, on obtient le 4-(2-chloro-4-méthoxyphényl)-5-méthyl-2-[N-(3,5-diméthylphényl)-N-propylamino]thiazole, avec les mêmes réactifs mais à température ordinaire en ce qui concerne la température de la réaction après introduction d'iodure de propyle.

Le produit obtenu est sous forme de poudre ; F = 40°C.

### EXEMPLE 13

### 4-(2-Chloro-4-méthoxyphényl)-5-méthyl-2-[N-(2-méthoxy-5-o-tolylphényl)-N-propyl amino]thiazole

(I) : R₁ = Cl , R₂ = OCH₃, R₃ = H ; R₄ = CH₃ ; R₅ = -CH₂CH₂CH₃ R₆ =

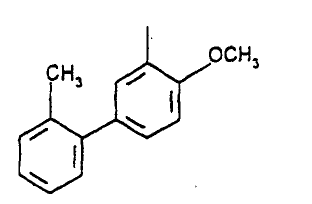

A 1 g de 4-(2-chloro-4-méthoxyphényl)-5-méthyl-2-[N-(5-bromo-2-méthoxyphényl)-N-propylamino]thiazole, en solution dans 25 ml d'éthanol et 0,8 ml d'eau, on ajoute successivement 1,96 g d'hydroxyde de barium, 0,52 g d'acide *o*-tolyl boronique et 40 mg de diacétate de palladium. Le mélange réactionnel est chauffé pendant 1 heure à reflux, de retour à température ambiante, il est filtré sur célite. Après évaporation du solvant, le résidu est repris au dichlorométhane. La phase organique est lavée à l'eau puis séchée sur sulfate de sodium. Le résidu d'évaporation est purifié sur gel de silice, éluant dichlorométhane/cyclohexane 9/1(v/v). Le produit attendu est obtenu sous forme de poudre ; F = 55°C.

### EXEMPLE 14

### 4-(2-Chloro-4-méthoxyphényl)-5-méthyl-2-[N-(2-méthoxy-5-nitrophényl)-N-propylamino]thiazole.

(I) : R₁ = Cl; R₂ = OCH₃ ; R₃ = H ; R₄ = CH₃ ; R₅ = -CH₂CH₂CH₃ R₆ =

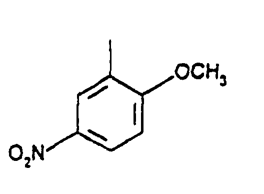

En procédant selon l'EXEMPLE 12 et en utilisant le 2-bromo-1-(2-chloro-4-méthoxyphényl) propan-1-one, *(Composé 1-2)* et la N-(2-méthoxy-5-nitrophényl)-N-propyl-thiourée, le produit attendu est obtenu sous forme de poudre ; F = 97°C.

### EXEMPLE 15

### 4-(2-Chloro-4-méthoxyphényl)-5-méthyl-2-[N-(5-amino-2-méthoxyphényl)-N-propylamino]thiazole.

(I) : R₁ = Cl ; R₂ = OCH₃ ; R₃ = H ; R₄ = CH₃ ; R₅ = -CH₂CH₂CH₃ R₆ =

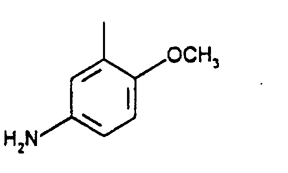

On met en solution 13,5 g de 4-(2-chloro-4-méthoxyphényl)-5-méthyl-2-[N-(2-méthoxy-5-nitrophényl)-N-propylamino]thiazole dans 120 ml de méthanol puis on ajoute 5,3 g de fer en poudre et 18,5 ml d'acide chlorhydrique concentré. On chauffe au reflux une demi-heure. Le méthanol est évaporé sous vide, le résidu est repris par du dichlorométhane avant d'être filtré sur célite. Le produit est extrait par de l'acide chlorhydrique 1 N. La phase acide est alors neutralisée par de l'hydroxyde de sodium avant d'être extraite par du dichlorométhane. Après évaporation du dichlorométhane, le brut réactionnel est purifié sur gel de silice, éluant dichlorométhane/méthanol 9515 (v/v).

Le produit attendu est solide ; F = 89°C.

### EXEMPLE 16

### 4-(2-Chloro-4-méthoxyphényl)-5-méthyl-2-[N-(5-acétamido-2-méthoxyphényl)-N-propylamino]thiazole

(I) : R₁ = Cl ; R₂ = OCH₃ ; R₃ = H ; R₄ = CH₃ ; R₅ = -CH₂CH₂CH₃ R₆ =

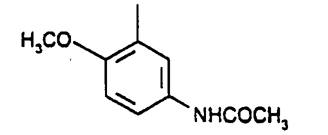

A 0°C, sous atmosphère inerte, 1 g de 4-(2-chloro-4-méthoxyphényl)-5-méthyl-2-[N-(5-amino-2-méthoxyphényl)-N-propylamino]thiazole est mis en solution dans 3,5 ml de dichlorométhane. On ajoute 0,4 ml de triéthylamine puis 0,14 ml de bromure d'acétyle. Après 1 heure à température ambiante, le mélange réactionnel est dilué au dichlorométhane. La phase organique est lavée à l'eau puis séchée sur sulfate de sodium, Le résidu d'évaporation est purifié sur gel de silice. éluant cyclohexane/acétate d'éthyle 1/1

Le produit se solidifie dans le pentane ; R = 71 % ; F =171°C.

### EXEMPLE 17

### 4-(2,4,6-Trichlorophényl)-5-méthyl-2-[N-(2, 5-ditrifluorométhylphényl)-N-propylamino]thiazole

(I) : R₁ = Cl ; R₂ = Cl ; R₃ = Cl ; R₄ = CH₃ ; R₅ = -CH₂CH₂CH₃ R₆ =

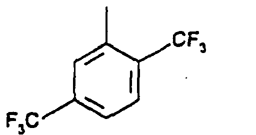

**Etape 1 :** en procédant selon l'EXEMPLE 2, en utilisant le 2-bromo-1-(2,4,6-trichlorophényl)éthan-1-one *(Composé 3-1)* et la N-(2,5-ditrifluorométhylphényl)-N-propylthiourée, on obtient le 4-(2,4,6-trichlorophényl)-2-[N-(2,5-ditrifluorométhyl-phényl)-N-propylamino]thiazole ; R = 33 %.
RMN ¹H (CDCl₃) : 0,87(*t*,3H, J = 3,8) ; 1,77(sext., 2H, J = 3,8, J = 7,8) ; 3,88 (m, 2H) ; 6,51(*s*,1H) ; 7,36(*s*,2H).

**Etape 2 :** le produit obtenu précédemment est mis en solution dans 50 ml de dichlorométhane. On ajoute 0,3 ml de triéthylamine suivi de 0,11 ml de brome. Le mélange est agité pendant 8 heures, une solution de thiosulfate de sodium est ajoutée. La phase aqueuse est extraite au dichlorométhane. Les phases organiques sont rassemblées, lavées à l'eau, séchées sur sulfate de sodium. Le résidu d'évaporation est purifié *sur* silice, éluant dichlorométhane/cyclohexane 1/2.
RMN ¹H (CDCl₃) : 0,92(*t*,3H, J = 3,7) ; 1,73 (sext., 2H, J = 3,7, J = 7,7) ; 3,71(*m*,2H) ; 7,38(*s*,2H) ; 7,72(*s*,1H) ; 7,80(*d*,1H, J = 4,2) ; 7,96(*d*,1H, J = 4,2).

**Etape 3 :** le produit issu de l'étape 2 (1,05 g) est mis en solution dans 40 ml de tétrahydrofurane. A - 70°C, sous atmosphère inerte, 1,6 ml de butyllithium (1,6 M dans l'hexane) est ajouté. Après 20 minutes, on ajoute 0,7 ml d'iodure de méthyl (6 equivalents). Le mélange revenu progressivement à température ambiante, est agité pendant 3 heures. Le mélange est alors hydrolysé à froid. La phase aqueuse est extraite au dichlorométhane, les phases organiques sont rassemblées, séchées sur sulfate de sodium. Le résidu d'évaporation est purifié sur gel de silice, éluant dichlorométhane/cyclohexane 1/2. Le produit est obtenu sous forme de poudre : F = 88°C.

En procédant selon les EXEMPLES 1 à 17, ci-dessus, on prépare de la même façon les EXEMPLES 18 à 75 décrits dans le TABLEAU I ci-après :

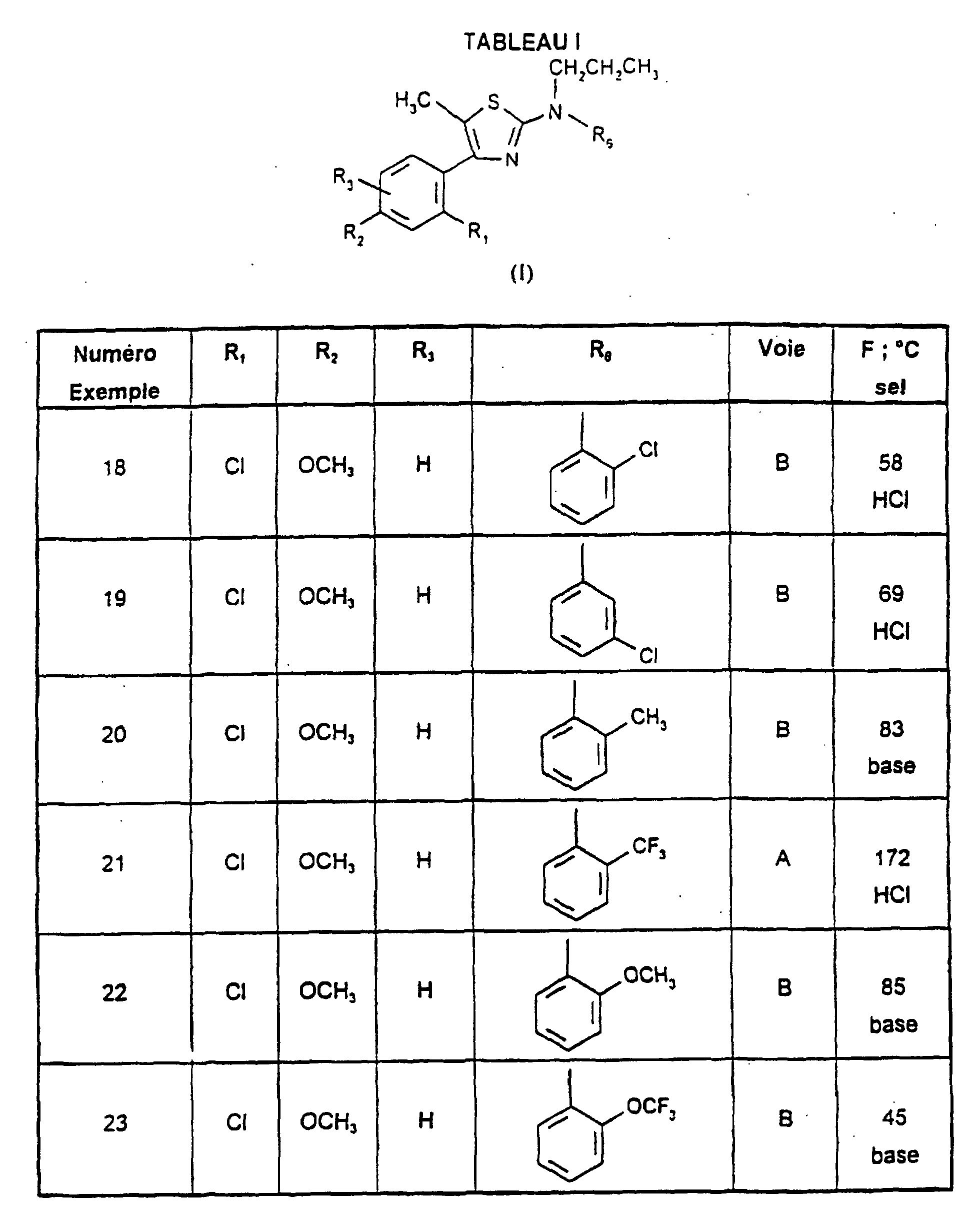

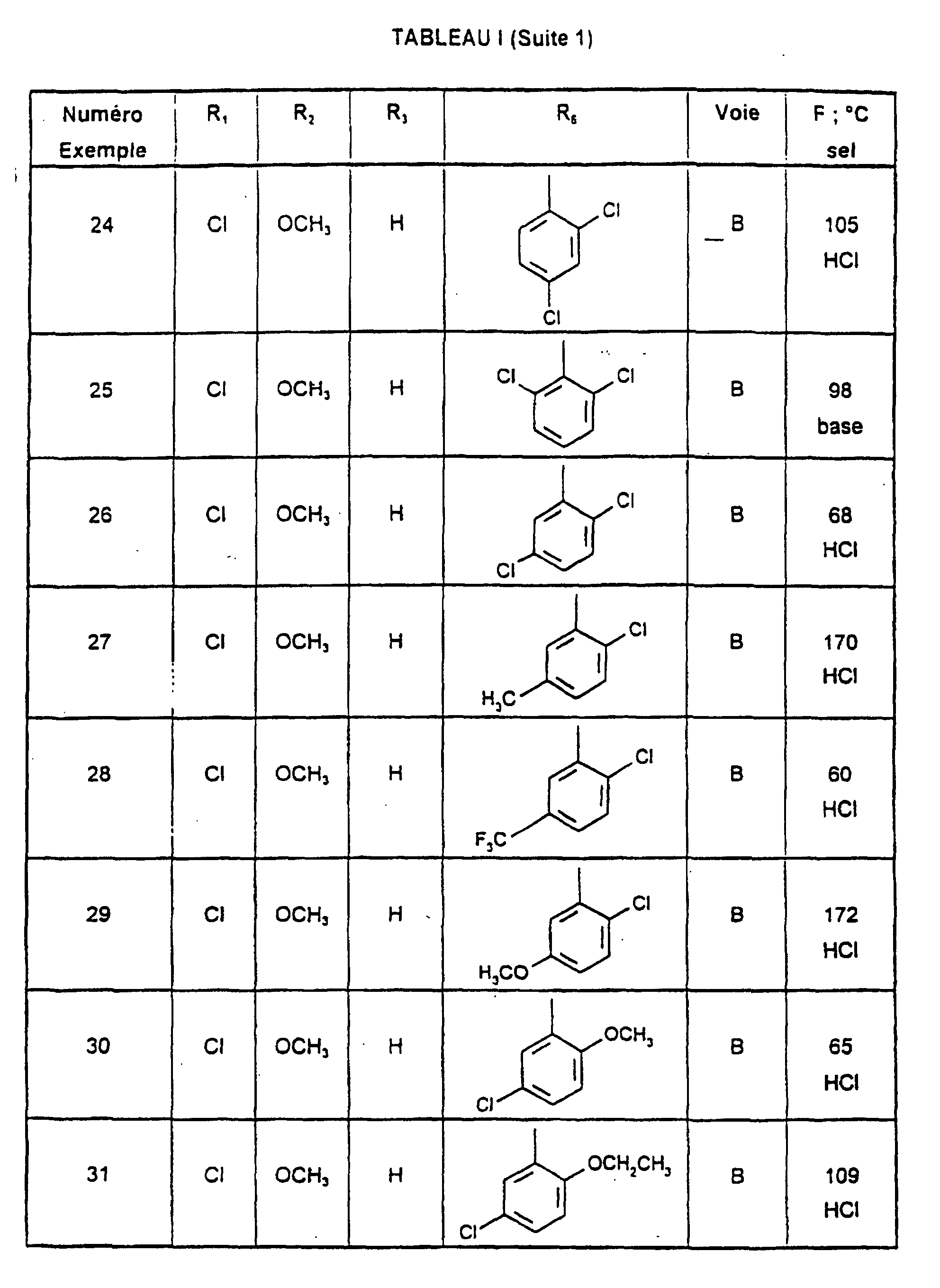

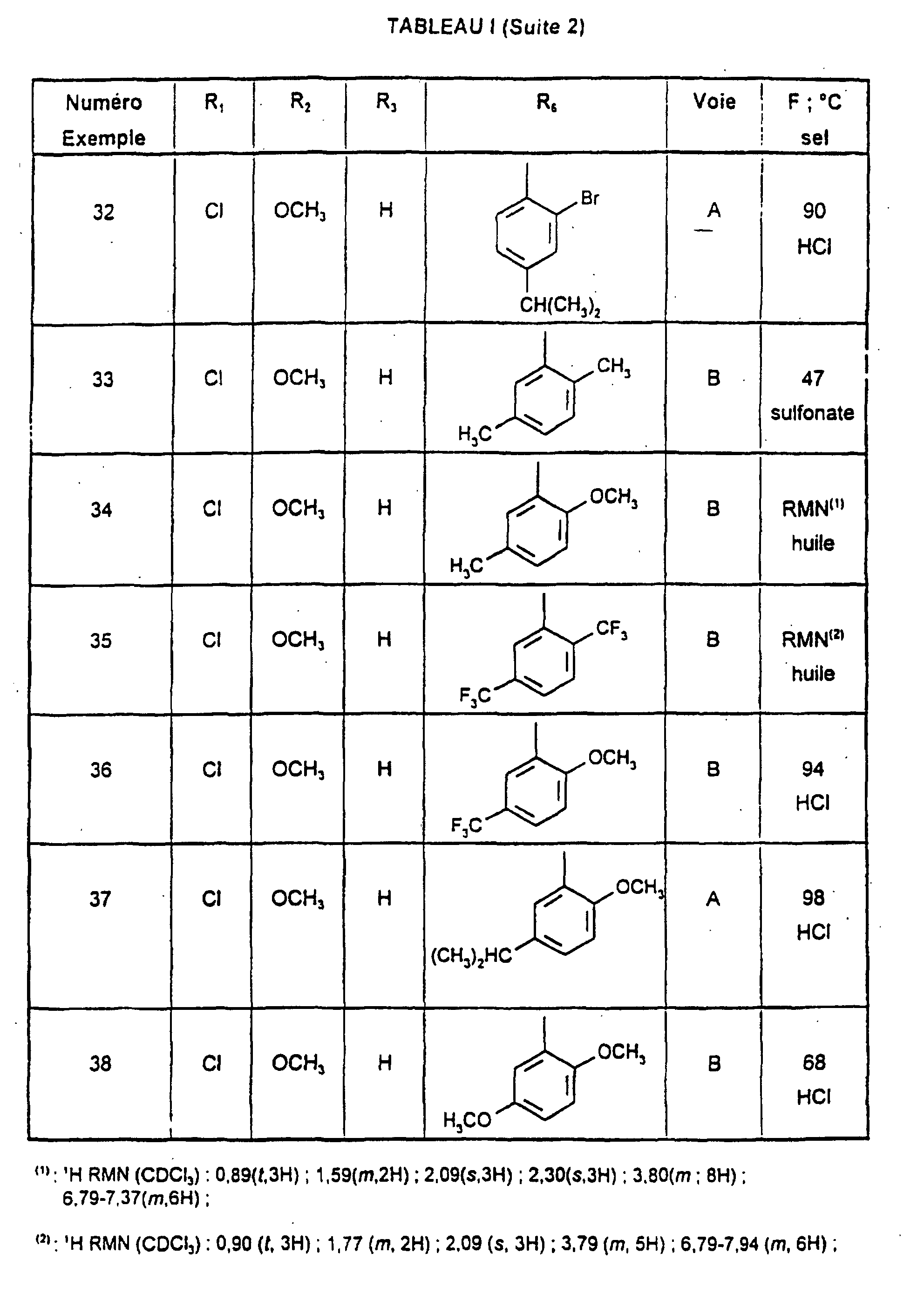

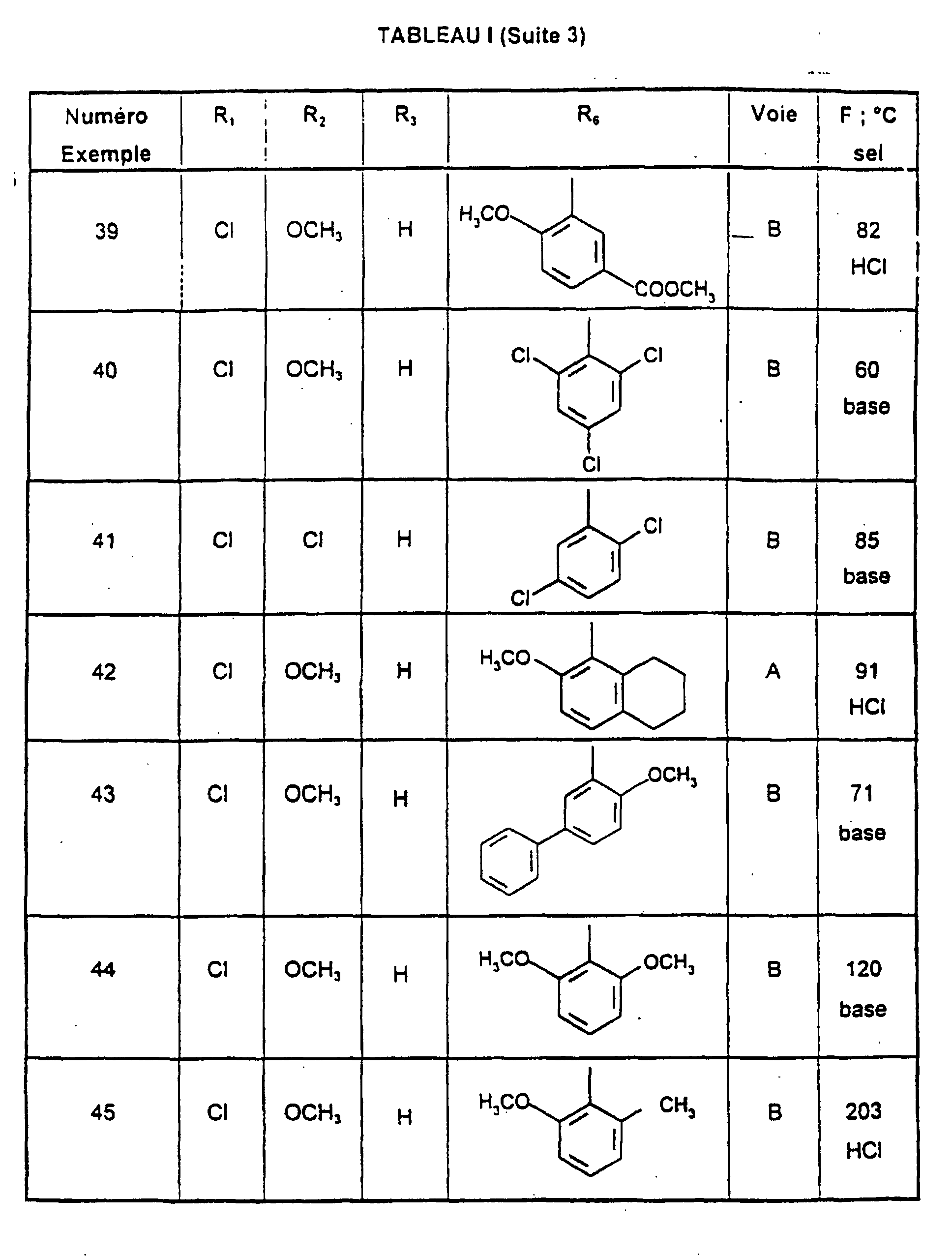

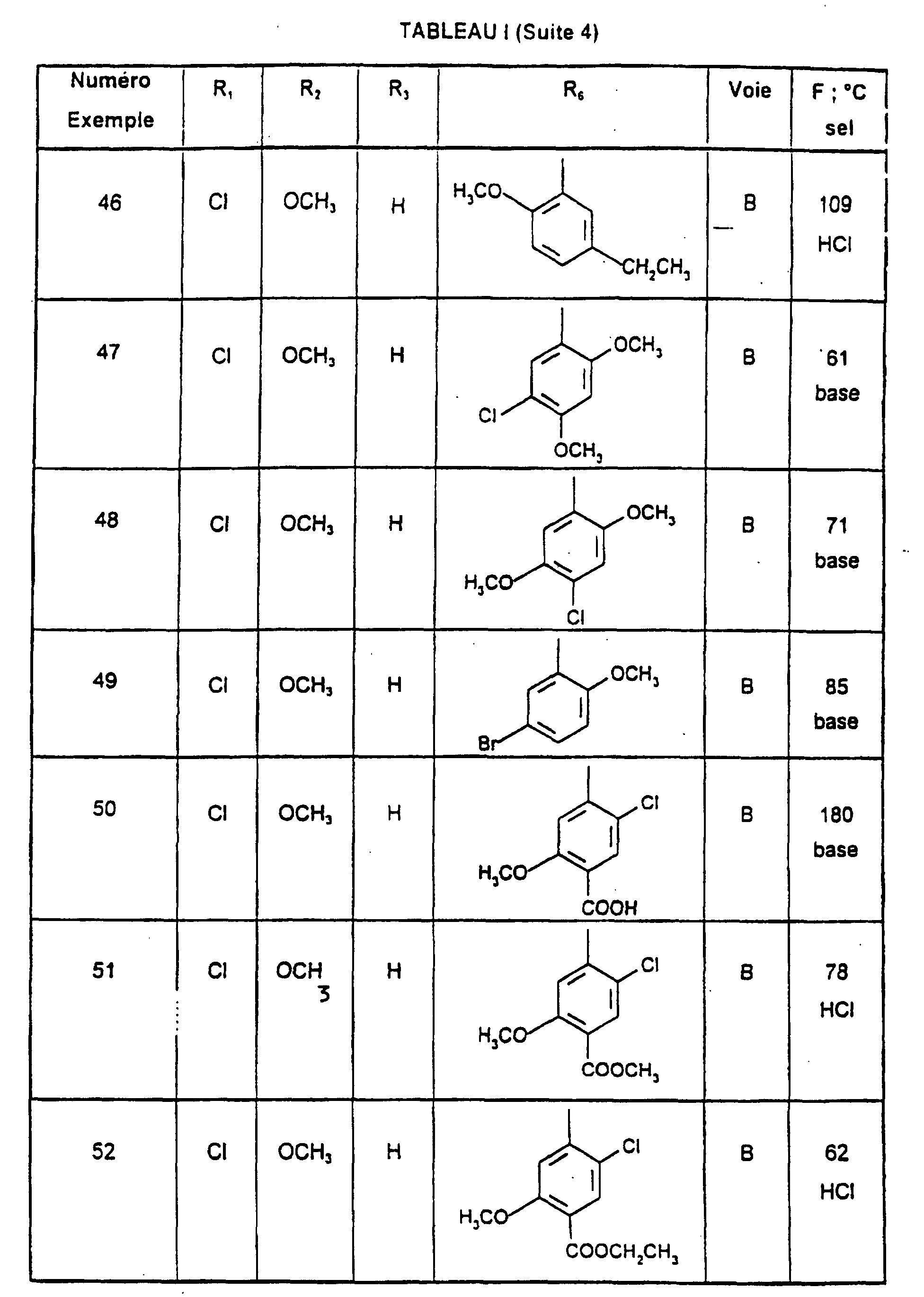

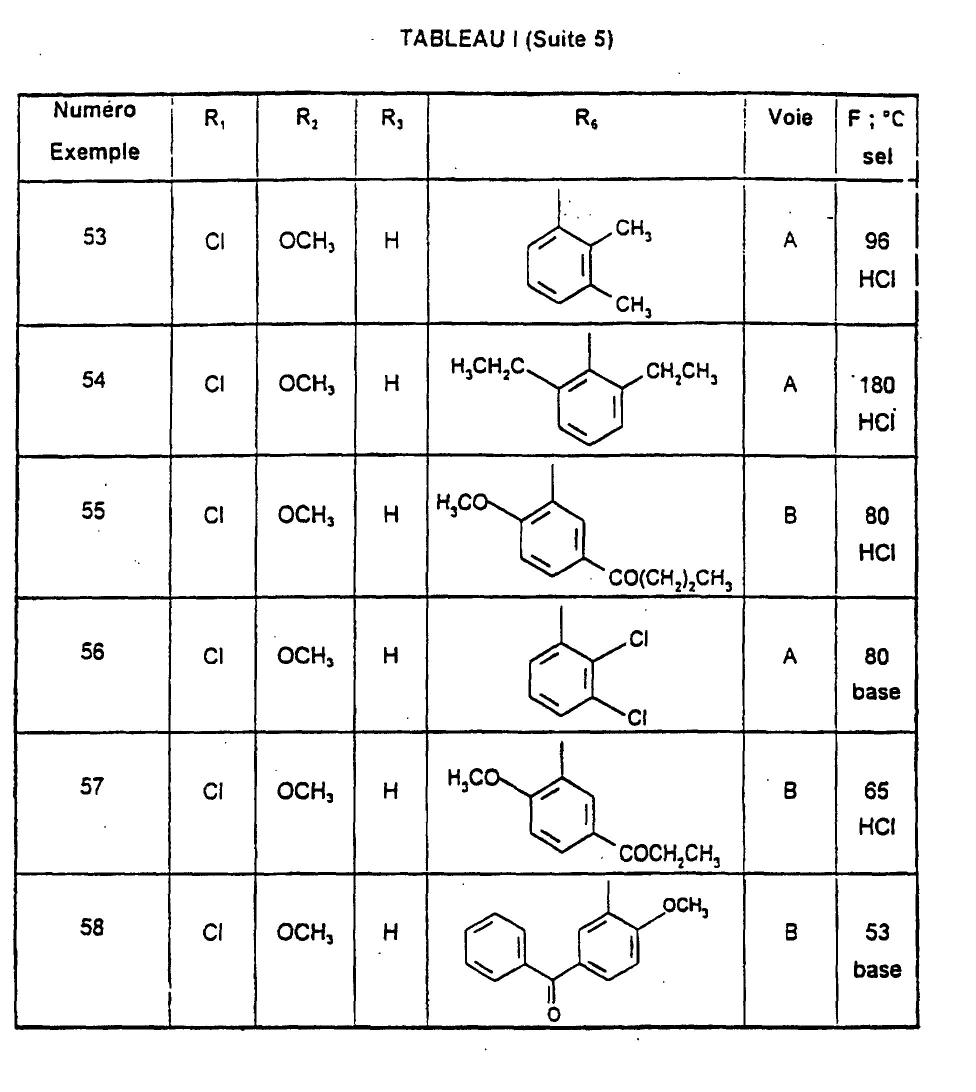

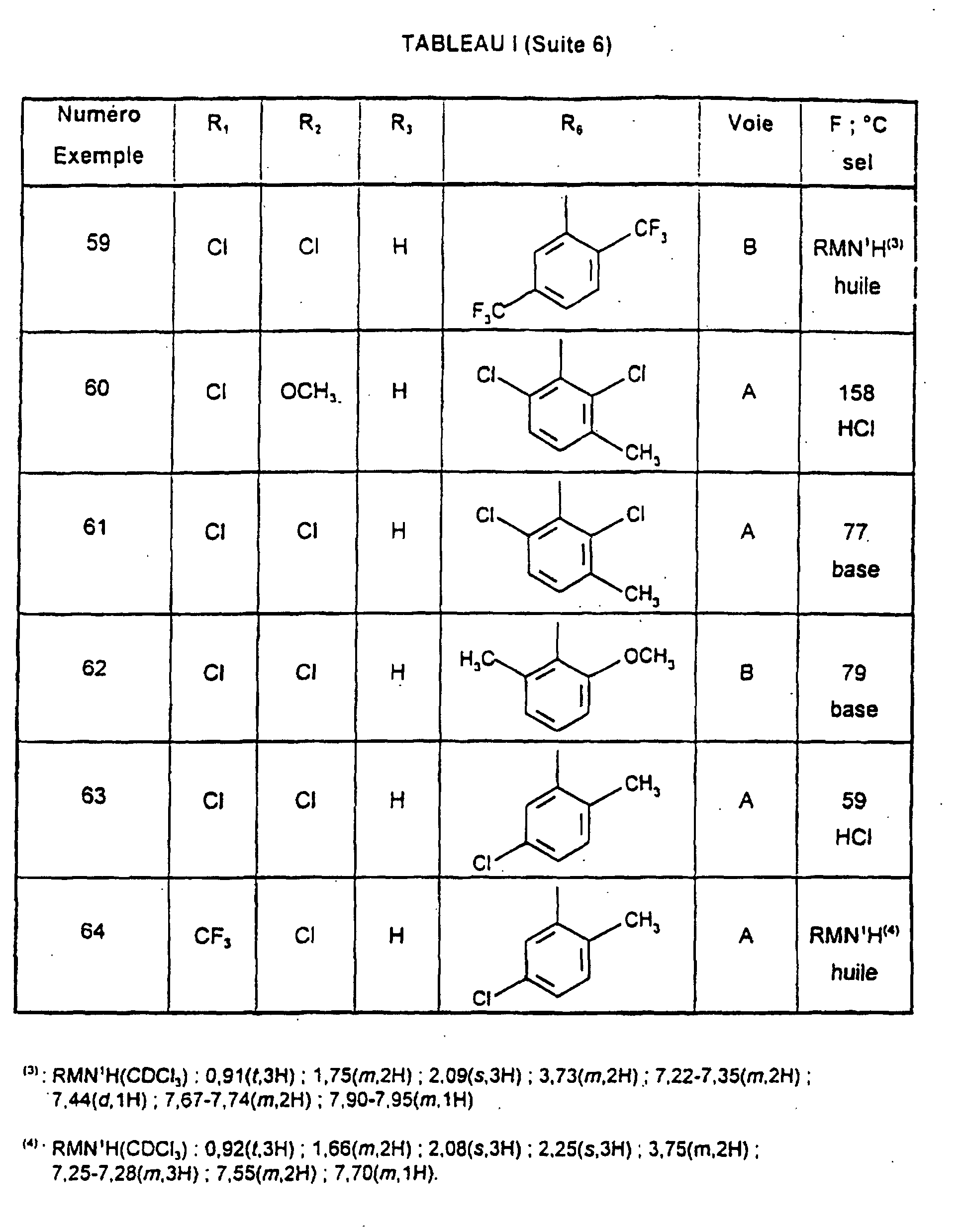

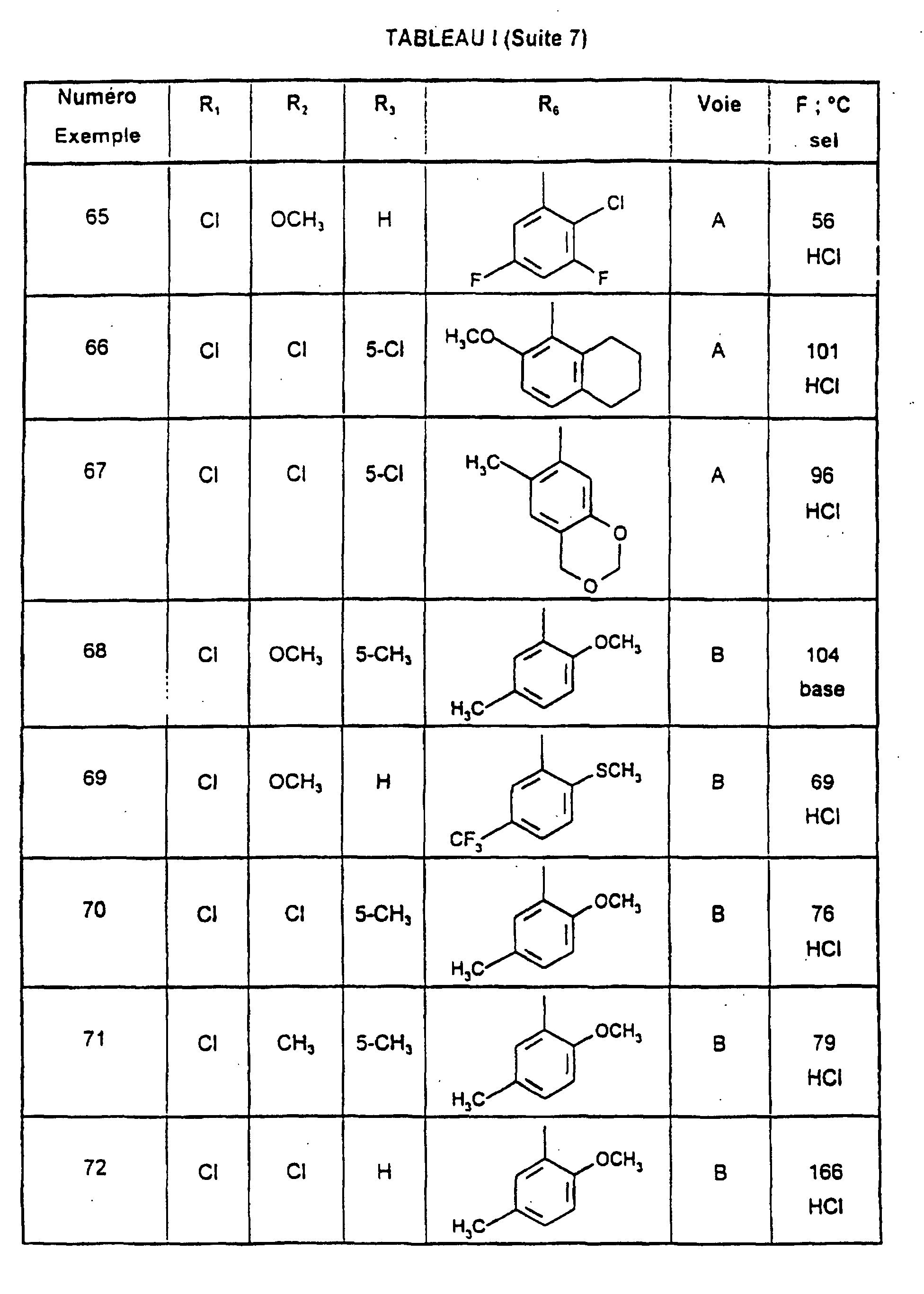

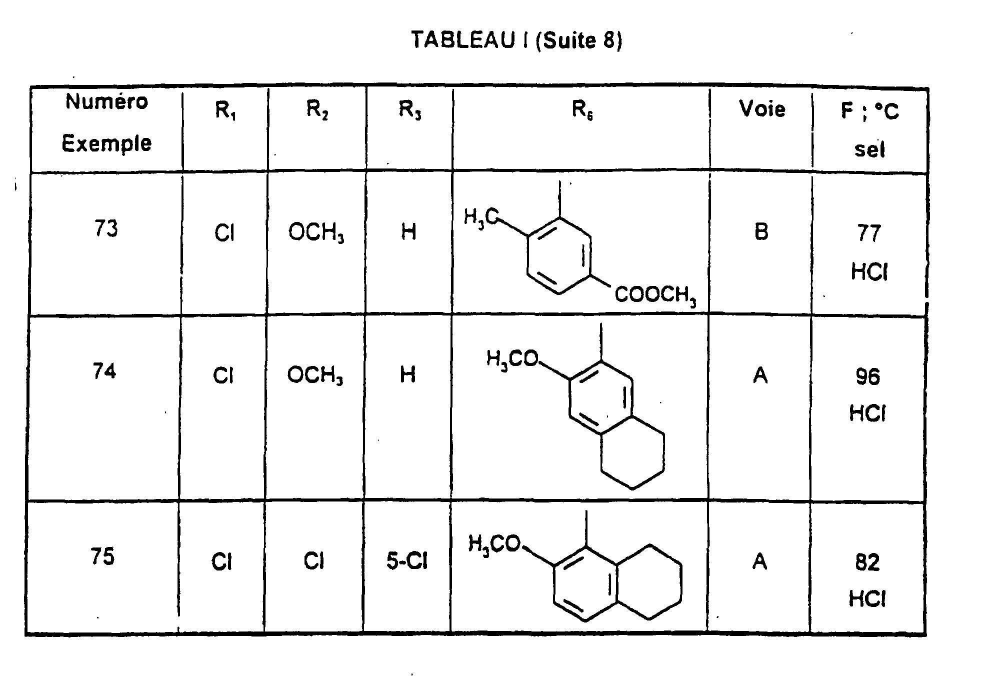

### EXEMPLE 76

### Bromhydrate de 4-(2-chloro-4-méthoxyphényl)-5-méthyl-2-[N-(5-chloro-2-méthylphényl)-N-prop-2-ynylamino]thiazole

(I) : R₁ = Cl ; R₂ = OCH₃ ; R₃ = H ; R₄ = CH₃ ; R₅ = -CH₂-C≡CH R₆ =

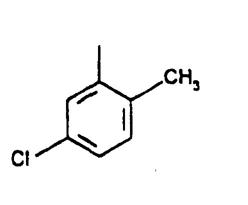

**Etape 1 :** En procédant selon l'EXEMPLE 11 mais en utilisant de l'iodure de prop-2-ynyle à l'étape 2, on obtient le 4-(2-chloro-4-méthoxyphényl)-5-méthyl-2-[N-(5-chloro-2-méthylphényl)amino]thiazole

**Etape 2 :** A 0°C, sous argon, 1,3 g du produit obtenu à l'étape 1 sont mis en solution dans 10 ml de diméthylformamide. On additionne 0,2 g d'hydrure de sodium (50 % dans l'huile). Après un quart d'heure, 0,8 ml d'une solution à 80 % de bromure de propargyle dans le toluène est ajoutée lentement. On agite le mélange réactionnel. Le mélange réactionnel est dilué avec de l'acétate d'éthyle et on l'hydrolyse.

La phase organique est lavée 3 fois à l'eau, puis séchée sur le sulfate de sodium. Après évaporation, le produit est purifié sur gel de silice, éluant cyclohexane, mélange dichlorométhane/acétate d'éthyle 80/20 (v/v). Le bromhydrate est obtenu, en ajoutant le produit obtenu précédemment en solution dans du dichlorométhane à une solution 0,2-M d'acide bromhydrique dans l'isopropanol. Après évaporation, obtention d'une poudre blanche ; F = 101°C.

## Revendications

1. Composé de formule : 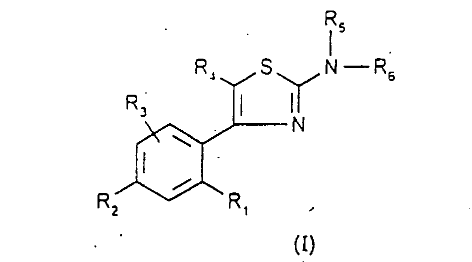 dans laquelle :
- R₁ et R₂ identiques ou différents représentent chacun indépendamment un atome d'halogène ; un (C₁-C₅)hydroxyalkyle ; un (C₁-C₅) alkyle ; un aralkyle dans lequel la partie aryle est en (C₆-C₈) et la partie alkyle en (C₁-C₄) ; un (C₁-C₅)alcoxy ; un groupe trifluorométhyle ; un groupe nitro ; un groupe nitrile ; un groupe -SR dans lequel R représente l'hydrogène, un (C₁-C₅)alkyle ou un aralkyle dans lequel la partie aryle est en (C₆-C₈) et la partie alkyle en (C₁-C₄) ; un groupe -S-CO-R^{o} dans lequel R^{o} représente un radical (C₁-C₅)alkyle ou un aralkyle dans lequel la partie aryle est en (C₅-C₈) et la partie alkyle est en (C₁-C₄) ; un groupe -COORa dans lequel Ra représente l'hydrogène ou un (C₁-C₅)alkyle ; un groupe -CONRaRb avec Ra et Rb tels que définis ci-dessus pour Ra ; un groupe -NRaRb avec Ra et Rb tels que définis précédemment pour Ra ; un groupe -CONRcRd ou -NRcRd dans lesquels Rc et Rd constituent avec l'atome d'azote auquel ils sont liés un hétérocycle de 5 à 7 chaînons ; ou un groupe -NHCO-NRaRb avec Ra et Rb tels que definis ci-dessus pour Ra ;
- R₃ représente l'hydrogène ou est tel que défini ci-dessus pour R₁ et R₂ ;
- R₄ représente un (C₁-C₅)alkyle ; un groupe hydroxyméthyle ; un groupe formyle ; ou un atome d'halogène ;
- R₅ représente un (C₁-C₅)alkyle ; un groupe cycloalkylalkyle dans lequel le cycloalkyle est en (C₃-C₇) et l'alkyle en (C₁-C₅) ; un alcényle de 3 à 6 atomes de carbone : un (C₁-C₅) hydroxyalkyle ; un groupe alkylcarbonyloxyalkyle dans lequel les alkyles sont en (C₁-C₅) ; ou un groupe alcynyle de 3 à 6 atomes de carbone ;
- R₆ représente un phényle substitué par un ou plusieurs substituants Z tel que défini ci-dessous; un groupe hétéroaromatique monocyclique de 5 à 7 chaînons substitué par un ou plusieurs radicaux Z tels que définis ci-dessous; ou un groupe bicyclique de 9 ou 10 chaînons constitué d'un monocycle aromatique comportant éventuellement un ou plusieurs hétéroatomes choisis parmi O, N et S condensé à un groupe cycloalkyle comportant éventuellement dans le cycle un ou plusieurs hétéroatomes choisis parmi O, N et S, lequel groupe bicyclique est substitué par un ou plusieurs substituants Z tels que définis ci-dessous et lequel est relié à l'azote par le cycle à caractère aromatique, étant entendu que R₆ ne représente pas un indane substitué et que le substituant Z représente un radical choisi parmi : un atome d'halogène, un groupe nitro, un groupe hydroxy, un groupe trifluorométhyle, un (C₁-C₅)alkyle, un (C₁-C₅)thioalkyle, un groupe -NRaRb avec Ra et Rb tels que définis précédemment pour Ra, un (C₁-C₅)hydroxyalkyle, un (C₁-C₅)alcoxy, un groupe trifluorométhyloxy, un alcoxyalkyle dans lequel les alkyles sont en (C₁-C₅), un groupe -COORa avec Ra tel que défini précédemment, un groupe -CONRaRb avec Ra et Rb tels que définis précédemment pour Ra, un carboxyl(C₁-C₅)alkyle, un alcoxycarbonylalkyle dans lequel les alkyles sont en (C₁-C₅), un (C₁-C₅)alkylcarbonyle, un alkylcarbonylalkyle dans lequel les alkyles sont en (C₁-C₅), un groupe morpholinocarbonyle ou morpholinocarbonyl(C₁-C₅)alkyle, ou un groupe -NRaCOORb avec Ra et Rb tels que définis précédemment, un groupe -NHCORₑ dans lequel Rₑ représente un (C₁-C₈)alkyle, un cycloalkylcarbonyle dans lequel le cycloalkyle est en (C₃-C₆), un cycloalkylalkylcarbonyle dans lequel le cycloalkyle est en (C₃-C₆) et l'alkyle en (C₁-C₃), un benzoyle, un phényle non substitué ou substitué par un (C₁-C₅)alkyle, par un (C₁-C₅)alcoxy, par un atome d'halogène, par un groupe nitro, par un groupe hydroxy ou par un groupe trifluorométhyle;
leurs stéréoisomères, leurs sels d'addition, leurs hydrates et/ou leurs solvates.

2. Composé de formule (I), selon la revendication 1, dans laquelle R₆ représente un groupe phényle ou tétrahydronaphtyle substitué par un ou plusieurs substituants Z tels que définis pour (I), R₁, R₂, R₃, R₄ et R₅ étant également tels que définis pour (I), un de leurs stéréoisomères, un de leurs sels, un de leurs hydrates et/ou un de leurs solvates.

3. Composé de formule (I), selon les revendications 1 et 2, dans laquelle R₄ représente un méthyle, R₁, R₂, R₃ et R₅ étant tels que définis pour (I), un de leurs stéréoisomères, un de leurs hydrates et/ou un de leurs solvates.

4. Composé de formule (I) selon la revendication 3, dans laquelle R₁ et/ou R₂ représente un halogène, un trifluorométhyle, un (C₁-C₅)alkyle ou un (C₁-C₅)alcoxy, R₄ représente un méthyle, R₆ représente un phényle au moins substitué en position 2 par un substituant Z tel que défini pour (I), R₃ et R₅ sont tels que définis pour (I), un de leurs stéréoisomères, un de leurs sels, un de leurs hydrates et/ou un de leurs solvates.

5. Composé selon la revendication 1 choisi parmi :
. 4-(2-Chloro-4-méthoxyphényl)-5-méthyl-2-[N-(5-éthoxycarbonyl-2-méthoxyphényl)-N-propylamino]thiazole
. 4-(2-Chloro-4-méthoxyphényl)-5-méthyl-2-[N-(5-chloro-2-méthylphényl)-N-propyl-amino]thiazole
. 4-(2-Chloro-4-méthoxyphényl)-5-méthyl-2-[N-(2-trifluorométhylphényl)-N-propyl-amino]thiazole
. 4-(2-Chloro-4-méthoxyphényl)-5-méthyl-2-[N-(2-méthoxyphényl)-N-propylamino] thiazole
. 4-(2-Chloro-4-méthoxyphényl)-5-méthyl-2-[N-(2,6-dichlorophényl)-N-propylamino] thiazole
. 4-(2-Chloro-4-méthoxyphényl)-5-méthyl-2-[N-(2,5-dichlorophényl)-N-propylamino] thiazole
. 4-(2-Chloro-4-méthoxyphényl)-5-méthyl-2-[N-(2-chloro-5-méthylphényl)-N-propyl-amino]thiazole
. 4-(2-Chloro-4-méthoxyphényl)-5-méthyl-2-[N-(2-chloro-5-trifluorométhylphényl)-N-propylamino]thiazole
. 4-(2-Chloro-4-méthoxyphényl)-5-méthyl-2-[N-(2-chloro-5-méthoxyphényl)-N-propylamino]thiazole
. 4-(2-Chloro-4-méthoxyphényl)-5-méthyl-2-[N-(5-chloro-2-méthoxyphényl)-N-propylamino]thiazole
. 4-(2-Chloro-4-méthoxyphényl)-5-méthyl-2-[N-(5-chloro-2-éthoxyphényl)-N-propylamino]thiazole
. 4-(2-Chloro-4-méthoxyphényl)-5-méthyl-2-[N-(2-méthoxy-5-méthylphényl)-N-propylamino]thiazole
. 4-(2-Chloro-4-méthoxyphényl)-5-méthyl-2-[N-(2,5-diméthylphényl)-N-propylamino]thiazole
. 4-(2-Chloro-4-méthoxyphényl)-5-méthyl-2-[N-(2,5-ditrifluorométhylphényl)-N-propylamino]thiazole
. 4-(2-Chloro-4-méthoxyphenyl)-5-méthyl-2-[N-(2-méthoxy-5-trifluorométhylphényl)-N-propylamino]thiazole
. 4-(2-Chloro-4-méthoxyphényl)-5-méthyl-2-[N-(2,5-diméthoxyphényl)-N-propylamino]thiazole
. 4-(2-Chloro-4-méthoxyphényl)-5-méthyl-2-[N-(2-méthoxy-5-méthoxycarbonylphényl)-N-propylamino]thiazole
. 4-(2,4-Dichlorophényl)-5-méthyl-2-[N-(2,5-dichlorophényl)-N-propylamino]thiazole
. 4-(2-Chloro-4-méthoxyphényl)-5-méthyl-2-[N-(5-acétyl-2-méthoxyphényl)-N-propylamino]thiazole
. 4-(2-Chloro-4-méthoxyphényl)-5-méthyl-2-[N-(2-(méthoxy)-5-(phényl)phényl)-N-propylamino]thiazole
. 4-(2-Chloro-4-méthoxyphényl)-5-méthyl-2-[N-(2,6-diméthoxyphényl)-N-propylamino]thiazole
. 4-(2-Chloro-4-méthoxyphényl)-5-méthyl-2-[N-(2-méthoxy-6-méthylphényl)-N-propylamino]thiazole
. 4-(2,4-dichlorophényl)-5-méthyl-2-[N-(2-méthoxy-6-méthylphényl)-N-propylamino]thiazole
. 4-(2-Chloro-4-méthoxyphényl)-5-méthyl-2-[N-(5-éthyl-2-méthoxyphényl)-N-propylamino]thiazole
. 4-(2-Chloro-4-méthoxyphényl)-5-méthyl-2-[N-(5-bromo-2-méthoxyphényl)-N-propylamino]thiazole
. 4-(2-Chloro-4-méthoxyphényl)-5-méthyl-2-[N-(2-méthoxy-5-o-tolylphényl)-N-propylamino]thiazole
. 4-(2,4,6-Trichlorophényl)-5-méthyl-2-[N-(2,5-ditrifluorométhylphényl)-N-propylamino]thiazole
. 4-(2,4-Dichlorophényl)-5-méthyl-2-[N-(2,5-ditrifluorométhylphényl)-N-propylamino]thiazole
. 4-(2-Chloro-4-méthoxyphényl)-5-méthyl-2-[N-(2-méthoxy-5-nitrophényl)-N-propylamino]thiazole
. 4-(2,4-Dichlorophényl)-5-méthyl-2-(N-(2,6-dichloro-5-méthylphényl)-N-propylamino]thiazole
. 4-(2,4-Dichlorophényl)-5-méthyl-2-[N-(2-méthoxy-6-méthylphényl)-N-propylamino]thiazole
. 4-(2,4-Dichlorophényl)-5-méthyl-2-[N-(5-chloro-2-méthylphényl)-N-propylamino]thiazole
. 4-(4-Chloro-2-trifluorométhylphényl)-5-méthyl-2-[N-(5-chloro-2-méthylphényl)-N-propylamino]thiazole
. 4-(2-Chloro-4-méthoxy-5-méthylphényl)-5-méthyl-2-[N-(2-méthoxy-5-méthylphényl)-N-propylamino]thiazole
. 4-(2-Chloro-4-méthoxyphényl)-5-méthyl-2-[N-(2-méthylthio-5-trifluorométhylphényl)-N-propylamino]thiazole
. 4-(2,4-Dichloro-5-méthylphenyl)-5-méthyl-2-[N-(2-méthoxy-5-méthylphényl)-N-propylamino]thiazole
. 4-(2-Dichloro-4,5-diméthylphényl)-5-méthyl-2-[N-(2-méthoxy-5-méthylphényl)-N-propylamino]thiazole
. 4-(2-Chloro-4-methoxyphényl)-5-méthyl-2-[N-(2-méthoxy-5-méthoxycarbonylphényl) -N-propylamino]thiazole
. 4-(2-Chloro-4-méthoxyphényl)-5-méthyl-2-[N-(5-chloro-2-méthylphényl)-N-prop-2-ynylamino]thiazole
un de leurs stéréoisomères, un de leurs sels, un de leurs hydrates et/ou un de leurs solvates.

6. Procédé de préparation des composés de formule (I), selon la revendication 1, **caractérisé en ce que** l'on fait réagir un dérivé alphahalogéné de formule (III) 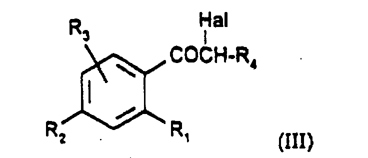 dans laquelle R₁, R₂, R₃ et R₄ sont tels que définis pour (I) et Hal représente un atome d'halogène de préférence de brome ou de chlore
soit avec une thiourée (VOIE A) de formule : 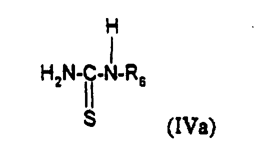 dans laquelle R₆ est tel que défini pour (I) pour obtenir un composé de formule (II) 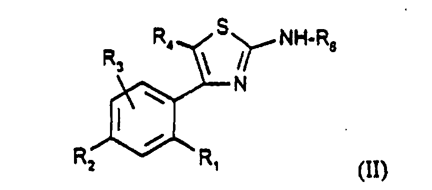 dans laquelle R₁, R₂, R₃, R₄ et R₆ sont tels que définis pour (I) pour le soumettre ensuite à une réaction d'alkylation pour fournir le composé (I),
soit avec une thiourée (VOIE B) de formule 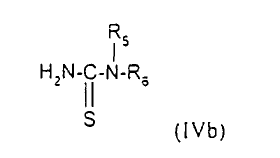 dans laquelle R₅ et R₆ sont tels que définis pour (I) pour conduire directement au composé (I),
et le cas échéant, les composés de formule (I) ainsi obtenus sont ensuite éventuellement séparés en leurs stéréoisomères possibles et/ou salifiés pour former les sels correspondants.

7. Utilisation des composés de formule (I), selon l'une quelconque des revendications 1 à 5 pour la préparation d'un médicament utilisable dans le traitement des maladies nécessitant une modulation de l'action du facteur de libération de l'hormone corticotrope.

8. Composition pharmaceutique contenant comme principe actif au moins un composé selon l'une quelconque des revendications 1 à 5 sous forme de base ou sous forme de sel avec un acide minéral ou organique pharmaceutiquement acceptable, en association ou en mélange avec un excipient inerte, non toxique pharmaceutiquement acceptable.

9. Composition pharmaceutique selon la revendication 8, sous forme d'unités de dosage, dans laquelle le principe actif est mélangé à au moins un excipient pharmaceutique.

10. Composition selon la revendication 9 dans laquelle chaque unité de dosage contient de 0,5 à 800 mg de principe actif.

11. Composition selon la revendication 10 dans laquelle chaque unité de dosage contient de 0,5 à 200 mg de principe actif.

12. Composition pharmaceutique contenant un composé selon les revendications 1 à 5 en association avec un autre principe actif anxiolytique, antidépresseur ou anorexigène.

13. Composé selon la revendication 1 dans laquelle R₆ représente un groupe hétéroaromatique monocyclique choisi parmi :
- azépine, pyridyle, pyrazinyle, pyridazinyle, pyrimidyle, triazinyle, furyle et thiényle.

14. Composé selon la revendication 1 dans laquelle R₆ représente un groupe bicyclique choisi parmi :
- 1,2,3,4-tétrahydroquinolyle, 1,2,3,4-tétrahydroisoquinolyle, 1,2,3,4-tétrahydrophtalazinyle, 1,2,3,4-tétrahydroquinazolinyle, 1,2,3,4-tétrahydroquinoxalinyle, 1,2,3,4-tétrahydrocinnolinyle, 1,2,3,4-tétrahydrobenzo-triazinyle, chromanyle, isochromanyle, indolinyle, isoindolinyle, 2,3-dihydroindazyle, 2,3-dihydrobenzoimidazolyle, 1,2-dihydrobenzotriazolyle, 2,3-dihydrobenzofurannyle, 2,3-dihydrobenzothiényle, 2,3-dihydrobenzoisothiazolyle, 2,3-dihydrobenzothiazolyle, 2,3-dihydrobenzoisoxazolyle, 2,3-dihydrobenzoxazolyle, 1,2-dihydrobenzoxazinyle, 1,2,3,4-tétrahydroptéridinyl, 8,9-dihydropurinyle.

## Claims

1. Compound of formula: 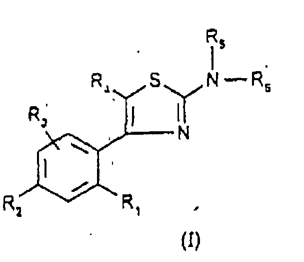 wherein:
- R₁ and R₂ which may be identical or different each represent, independently of one another, a halogen atom; a (C₁₋₅)hydroxyalkyl; a (C₁₋₅)alkyl; an aralkyl wherein the aryl moiety is (C₆₋₈) and the alkyl part is (C₁₋₄) ; a (C₁₋₅)alkoxy; a trifluoromethyl group; a nitro group; a nitrile group; a group -SR wherein R denotes hydrogen, a (C₁₋₅)alkyl or an aralkyl wherein the aryl part is (C₅₋₈)and the alkyl part is (C₁₋₄); a group -S-CO-R^{o} wherein R^{o} denotes a (C₁₋₅)alkyl radical or an aralkyl wherein the aryl part is (C₅₋₈)and the alkyl part is (C₁₋₄); a group -COORa wherein Ra denotes hydrogen or a (C₁₋₅)alkyl; a group -CONRaRb wherein Ra and Rb are as hereinbefore defined for Ra; a group -NRaRb wherein Ra and Rb are as hereinbefore defined for Ra; a group -CONRcRd or -NRcRd wherein Rc and Rd together with the nitrogen atom to which they are linked form a 5- to 7-membered heterocyclic group; or a group -NHCO-NRaRb wherein Ra and Rb are as hereinbefore defined for Ra;
- R₃ denotes hydrogen or is as defined for R₁ and R₂ hereinbefore;
- R₄ denotes a (C₁₋₅)alkyl; a hydroxymethyl group; a formyl group; or a halogen atom;
- R₅ denotes a (C₁₋₅)alkyl; a cycloalkylalkyl group wherein the cycloalkyl group is (C₃₋₇) and the alkyl is (C₁₋₅); an alkenyl group having 3 to 6 carbon atoms; a (C₁₋₅)hydroxyalkyl group; an alkylcarbonyloxyalkyl group wherein the alkyls are (C₁₋₅); or an alkynyl group having 3 to 6 carbon atoms;
- R₆ denotes a phenyl substituted by one or more substituents Z as defined hereinafter; a monocyclic 5-7-membered heteroaromatic group substituted by one or more radicals Z as defined hereinafter; or a 9- or 10-membered bicyclic group consisting of an aromatic monocyclic group optionally having one or more heteroatoms selected from among O, N and S, fused to a cycloalkyl group optionally comprising in the ring one or more heteroatoms selected from among O, N and S, said bicyclic group being substituted by one or more substituents Z as defined hereinafter and which is connected to the nitrogen by the aromatic ring, given that R₆ does not denote a substituted indane and the substituent Z denotes a radical selected from among: a halogen atom, a nitro group, a hydroxy group, a trifluoromethyl group, a (C₁₋₅)alkyl, a (C₁₋₅) thioalkyl, a group -NRaRb wherein Ra and Rb are as hereinbefore defined for Ra, a (C₁₋₅)hydroxyalkyl, a (C₁₋₅)alkoxy, a trifluoromethyloxy group, an alkoxyalkyl wherein the alkyls are (C₁₋₅), a group -COORa wherein Ra is as hereinbefore defined, a group -CONRaRb wherein Ra and Rb are as hereinbefore defined for Ra, a carboxyl(C₁₋₅)alkyl, an alkoxycarbonylalkyl wherein the alkyls are (C₁₋₅), a (C₁₋₅)alkylcarbonyl, an alkylcarbonylalkyl wherein the alkyls are (C₁₋₅), a morpholinocarbonyl or morpholinocarbonyl (C₁₋₅) alkyl group, or a group -NRaCOORb wherein Ra and Rb are as hereinbefore defined, a group -NHCORₑ wherein Rₑ denotes a (C₁₋₈)alkyl, a cycloalkylcarbonyl wherein the cycloalkyl is (C₃₋₆), a cycloalkylalkylcarbonyl wherein the cycloalkyl is (C₃₋₆) and the alkyl is (C₁₋₃), a benzoyl, a phenyl which is unsubstituted or substituted by a (C₁₋₅)alkyl, by a (C₁₋₅)alkoxy, by a halogen atom, by a nitro group, by a hydroxy group or by a trifluoromethyl group;
the stereoisomers, the addition salts, the hydrates and/or the solvates thereof.

2. Compound of formula (I) according to claim 1, wherein R₆ denotes a phenyl or tetrahydronaphthyl group substituted by one or more substituents Z as defined for (I), R₁, R₂, R₃, R₄ and R₅ also being as defined for (I), one of the stereoisomers, one of the salts, one of the hydrates and/or one of the solvates thereof.

3. Compound of formula (I) according to claims 1 and 2, wherein R₄ denotes a methyl, R₁, R₂, R₃, R₄ and R₅ being as defined for (I), one of the stereoisomers, one of the hydrates and/or one of the solvates thereof.

4. Compound of formula (I) according to claim 3, wherein R₁ and/or R₂ denotes a halogen, a trifluoromethyl, a (C₁₋₅)alkyl or a (C₁₋₅)alkoxy, R₄ denotes a methyl, R₆ denotes a phenyl at least substituted in the 2 position by a substituent Z as defined for (I), R₃ and R₅ are as defined for (I), one of the stereoisomers, one of the salts, one of the hydrates and/or one of the solvates thereof.

5. Compound according to claim 1 selected from among:
- 4-(2-chloro-4-methoxyphenyl)-5-methyl-2-[N-(5-ethoxycarbonyl-2-methoxyphenyl)-N-propylamino]thiazole
- 4-(2-chloro-4-methoxyphenyl)-5-methyl-2-[N-(5-chloro-2-methylphenyl)-N-propylamino]thiazole
- 4-(2-chloro-4-methoxyphenyl)-5-methyl-2-[N-(2-trifluoromethylphenyl)-N-propylamino]thiazole
- 4-(2-chloro-4-methoxyphenyl)-5-methyl-2-[N-(2-methoxyphenyl)-N-propylamino]thiazole
- 4-(2-chloro-4-methoxyphenyl)-5-methyl-2-[N-(2,6-dichlorophenyl)-N-propylamino]thiazole
- 4-(2-chloro-4-methoxyphenyl)-5-methyl-2-[N-(2,5-dichlorophenyl)-N-propylamino]thiazole
- 4-(2-chloro-4-methoxyphenyl)-5-methyl-2-[N-(2-chloro-5-methylphenyl)-N-propylamino]thiazole
- 4-(2-chloro-4-methoxyphenyl)-5-methyl-2-[N-(2-chloro-5-trifluoromethylphenyl)-N-propylamino]thiazole
- 4-(2-chloro-4-methoxyphenyl)-5-methyl-2-[N-(2-chloro-5-methoxyphenyl)-N-propylamino]thiazole
- 4-(2-chloro-4-methoxyphenyl)-5-methyl-2-[N-(5-chloro-2-methoxyphenyl)-N-propylamino]thiazole
- 4-(2-chloro-4-methoxyphenyl)-5-methyl-2-[N-(5-chloro-2-ethoxyphenyl)-N-propylamino]thiazole
- 4-(2-chloro-4-methoxyphenyl)-5-methyl-2-[N-(2-methoxy-5-methylphenyl)-N-propylamino]thiazole
- 4-(2-chloro-4-methoxyphenyl)-5-methyl-2-[N-(2,5-dimethylphenyl)-N-propylamino]thiazole
- 4-(2-chloro-4-methoxyphenyl)-5-methyl-2-[N-(2,5-ditrifluoromethylphenyl)-N-propylamino]thiazole
- 4-(2-chloro-4-methoxyphenyl)-5-methyl-2-[N-(2-methoxy-5-trifluoromethylphenyl)-N-propylamino]thiazole
- 4-(2-chloro-4-methoxyphenyl)-5-methyl-2-[N-(2,5-dimethoxyphenyl)-N-propylamino]thiazole
- 4-(2-chloro-4-methoxyphenyl)-5-methyl-2-[N-(2-methoxy-5-methoxycarbonylphenyl)-N-propylamino]thiazole
- 4-(2,4-dichlorophenyl)-5-methyl-2-[N-(2,5-dichlorophenyl)-N-propylamino]thiazole
- 4-(2-chloro-4-methoxyphenyl)-5-methyl-2-[N-(5-acetyl-2-methoxyphenyl)-N-propylamino]thiazole
- 4-(2-chloro-4-methoxyphenyl)-5-methyl-2-[N-(2-(methoxy)-5-(phenyl)phenyl)-N-propylamino]thiazole
- 4-(2-chloro-4-methoxyphenyl)-5-methyl-2-[N-(2,6-dimethoxyphenyl)-N-propylamino]thiazole
- 4-(2-chloro-4-methoxyphenyl)-5-methyl-2-[N-(2-methoxy-6-methylphenyl)-N-propylamino]thiazole
- 4-(2,4-dichlorophenyl)-5-methyl-2-[N-(2-methoxy-6-methylphenyl)-N-propylamino]thiazole
- 4-(2-chloro-4-methoxyphenyl)-5-methyl-2-[N-(5-ethyl-2-methoxyphenyl)-N-propylamino]thiazole
- 4-(2-chloro-4-methoxyphenyl)-5-methyl-2-[N-(5-bromo-2-methoxyphenyl)-N-propylamino]thiazole
- 4-(2-chloro-4-methoxyphenyl)-5-methyl-2-[N-(2-methoxy-5-o-tolylphenyl)-N-propylamino]thiazole
- 4-(2,4,6-trichlorophenyl)-5-methyl-2-[N-(2,5-ditrifluoromethylphenyl)-N-propylamino]thiazole
- 4-(2,4-dichlorophenyl)-5-methyl-2-[N-(2,5-ditrifluoromethylphenyl)-N-propylamino]thiazole
- 4-(2-chloro-4-methoxyphenyl)-5-methyl-2-[N-(2-methoxy-5-nitrophenyl)-N-propylamino]thiazole
- 4-(2,4-dichlorophenyl)-5-methyl-2-[N-(2,6-dichloro-5-methylphenyl)-N-propylamino]thiazole
- 4-(2,4-dichlorophenyl)-5-methyl-2-[N-(2-methoxy-6-methylphenyl)-N-propylamino]thiazole
- 4-(2,4-dichlorophenyl)-5-methyl-2-[N-(5-chloro-2-methylphenyl)-N-propylamino]thiazole
- 4-(4-chloro-2-trifluoromethylphenyl)-5-methyl-2-[N-(5-chloro-2-methylphenyl)-N-propylamino]thiazole
- 4-(2-chloro-4-methoxy-5-methylphenyl)-5-methyl-2-[N-(2-methoxy-5-methylphenyl)-N-propylamino]thiazole
- 4-(2-chloro-4-methoxyphenyl)-5-methyl-2-[N-(2-methylthio-5-trifluoromethylphenyl)-N-propylamino]thiazole
- 4-(2,4-dichloro-5-methylphenyl)-5-methyl-2-[N-(2-methoxy-5-methylphenyl)-N-propylamino]thiazole
- 4-(2-dichloro-4,5-dimethylphenyl)-5-methyl-2-[N-(2-methoxy-5-methylphenyl)-N-propylamino]thiazole
- 4-(2-chloro-4-methoxyphenyl)-5-methyl-2-[N-(2-methoxy-5-methoxycarbonylphenyl)-N-propylamino]thiazole
- 4-(2-chloro-4-methoxyphenyl)-5-methyl-2-[N-(5-chloro-2-methylphenyl)-N-prop-2-ynylamino]thiazole,
one of the stereoisomers, one of the salts, one of the hydrates and/or one of the solvates thereof.

6. Process for preparing the compounds of formula (I) according to claim 1, **characterised in that** an alpha-halogenated derivative of formula (III) 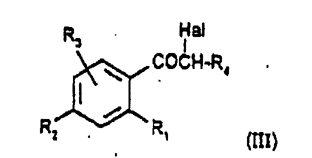 wherein R₁, R₂, R₃ and R₄ are as defined for (I) and Hal denotes a halogen atom, preferably bromine or chlorine, is reacted either with a thiourea (METHOD A) of formula: 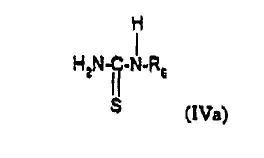 wherein R₆ is as defined for (I) to obtain a compound of formula (II) 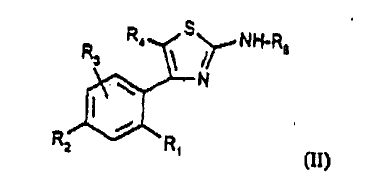 wherein R₁, R₂, R₃, R₄ and R₆ are as defined for (I), which is then subjected to an alkylation reaction to yield the compound (I),
or with a thiourea (METHOD B) of formula 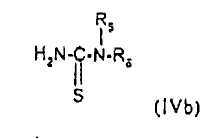 wherein R₅ and R₆ are as defined for (I), to lead directly to the compound (I),
and if appropriate the compounds of formula (I) thus obtained are then optionally separated into their possible stereoisomers and/or salified to form the corresponding salts.

7. Use of the compounds of formula (I) according to any one of claims 1 to 5 for preparing a medicament which can be used in the treatment of complaints requiring modulation of the action of the corticotropic hormone releasing agent.

8. Pharmaceutical composition containing as active principle at least one compound according to any one of claims 1 to 5 in the form of a base or in the form of a salt with a pharmaceutically acceptable inorganic or organic acid, in conjunction with or in admixture with a pharmaceutically acceptable inert non-toxic excipient.

9. Pharmaceutical composition according to claim 8, in the form of dosage units, in which the active principle is mixed with at least one pharmaceutical excipient.

10. Composition according to claim 9, wherein each dosage unit contains 0.5 to 800 mg of active principle.

11. Composition according to claim 10, wherein each dosage unit contains 0.5 to 200 mg of active principle.

12. Pharmaceutical composition containing a compound according to claims 1 to 5 in conjunction with an anxiolytic, antidepressant or anorexigenic active principle.

13. Compound according to claim 1 wherein R₆ denotes a monocyclic heteroaromatic group selected from among:
azxepine, pyridyl, pyrazinyl, pyridazinyl, pyrimidyl, triazinyl, furyl and thienyl.

14. Compound according to claim 1 wherein R₆ denotes a bicyclic group selected from among:
1,2,3,4-tatrahydroquinolyl, 1,2,3,4-tetrahydroisoquinolyl, 1,2,3,4-tetrahydrophthalazinyl, 1,2,3,4-tetrahydroquinazolinyl, 1,2,3,4-tetrahydroquinoxalinyl, 1,2,3,4-tetrahydrocinnolinyl, 1,2,3,4-tetrahydrobenzo-triazinyl, chromanyl, isochromanyl, indolinyl, isoindolinyl, 2,3-dihydroindazyl, 2,3-dihydrobenzoimidazolyl, 1,2-dihydrobenzotriazolyl, 2,3-dihydrobenzofuranyl, 2,3-dihydrobenzothienyl, 2,3-dihydrobenzoisothiazolyl, 2,3-dihydrobenzothiazolyl, 2,3-dihydrobenzoisoxazolyl; 2,3-dihydrobenzoxazolyl, 1,2-dihydrobenzoxazinyl, 1,2,3,4-tetrahydropteridinyl, 8,9-dihydropurinyl.

## Patentansprüche

1. Verbindung der Formel 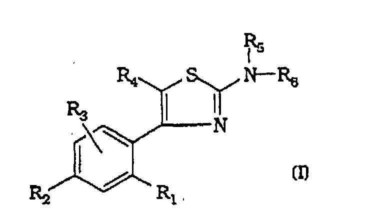 in der:
- R₁ und R₂, die gleichartig oder verschieden sind, jeweils unabhängig voneinander ein Halogenatom; (C₁-C₅)-Hydroxyalkyl; (C₁-C₅)-Alkyl; Aralkyl, worin der Arylrest (C₆-C₈) und der Alkylrest (C₁-C₄) ist; (C₁-C₅)-Alkoxy; eine Trifluormethylgruppe, eine Nitrogruppe, eine Nitrilgruppe; eine Gruppe -SR, worin R Wasserstoff, (C₁-C₅)-Alkyl oder Aralkyl bedeutet, worin der Arylrest (C₆-C₈) und der Alkylrest (C₁-C₄) ist; eine Gruppe -S-CO-R⁰, worin R⁰ eine (C₁-C₅)-Alkylgruppe oder Aralkylgruppe darstellt, worin der Arylrest (C₆-C₈) und der Alkylrest (C₁-C₄) ist; eine Gruppe -COORa, worin Ra Wasserstoff oder (C₁-C₅)-Alkyl darstellt; eine Gruppe -CONRaRb, worin Ra und Rb die oben für Ra angegebenen Bedeutungen besitzen; eine Gruppe -NRaRb, worin Ra und Rb die oben für Ra angegebenen Bedeutungen besitzen; eine Gruppe -CONRcRd oder -NRcRd, worin Rc und Rd mit dem Stickstoffatom, an das sie gebunden sind, einen Heterocyclus mit 5 bis 7 Kettengliedern bilden; oder eine Gruppe -NHCO-NRaRb, worin Ra und Rb die oben für Ra angegebenen Bedeutungen besitzen, bedeuten;
- R₃ Wasserstoff bedeutet oder die oben für R₁ und R₂ angegebenen Bedeutungen besitzt;
- R₄ (C₁-C₅)-Alkyl; eine Hydroxymethylgruppe; eine Formylgruppe; oder ein Halogenatom bedeutet;
- R₅ (C₁-C₅)-Alkyl; eine Cycloalkylalkylgruppe, worin der Cycloalkylrest (C₃-C₇) und der Alkylrest (C₁-C₅) ist; Alkenyl mit 3 bis 6 Kohlenstoffatomen; (C₁-C₅)-Hydroxyalkyl; eine Alkylcarbonyloxyalkylgruppe. worin die Alkyireste (C₁-C₅) sind; oder eine Alkinylgruppe mit 3 bis 6 Kohlenstoffatomen darstellt;
- R₆ Phenyl substituiert durch einen oder mehrere Substituenten 2, wie sie nachfolgend definiert sind; eine monocyclische heteroaromatische Gruppe mit 5 bis 7 Kettengliedern substituiert durch einen oder mehrere Reste Z, wie sie nachfolgend definiert sind; oder eine bicyclische Gruppe mit 9 oder 10 Kettengliedern gebildet aus einem aromatischen Monocyclus, der gegebenenfalls ein oder mehrere Heteroatome ausgewählt aus O, N und S aufweist, und kondenslert ist mit einer Cycloalkylgruppe, die gegebenenfalls im Ring ein oder mehrere Heteroatome ausgewählt aus O, N und S aufweist, welche bicyclische Gruppe substituiert ist durch einen oder mehrere Substituenten Z. wie sie nachfolgend definiert sind, und welche über den aromatischen Ring mit dem Stickstoff verbunden ist, mit der Maßgabe bedeutet, daß R₆ keinen substituierten Indanrest darstellt und der Substituent Z ausgewählt ist aus: Halogenatomen, Nitrogruppen, Hydroxygruppen. Trifluormethylgruppen, (C₁-C₅)-Alkylgruppen, (C₁-C₅)-Thioalkylgruppen, Gruppen -NRaRb, worin Ra und Rb die oben für Ra angegebenen Bedeutungen besitzen, (C₁-C₅)-Hydroxyalkylgruppen, (C₁-C₅)-Alkoxygruppen, Trifluormethyloxygruppen, Alkoxyalkylgruppen, worin die Alkylreste (C₁-C₅) sind, Gruppen -COORa, worin Ra die oben angegebenen Bedeutungen besitzt, Gruppen -COORaRb, worin Ra und Rb die oben für Ra angegebenen Bedeutungen besitzen, Carboxyl-(C₁-C₅)-alkylgruppen, Alkylcarbonylalkylgruppen, worin die Alkylreste (C₁-C₅) sind, Morpholinocarbonylgruppen oder Morpholinocarbonyl-(C₁-C₅)-alkylgruppen, oder Gruppen -NRaCOORb, worin Ra und Rb die oben angegebenen Bedeutungen besitzen, Gruppen -NHCOR_{c}, worin R_{c} (C₁-C₈)-Alkyl, Cycloalkylcarbonyl, worin der Cycloalkylrest (C₃-C₆) ist, Cycloalkylalkylcarbonyl, worin der Cycloalkylrest (C₃-C₆) und der Alkylrest (C₁-C₃) ist, Benzoyl, Phenyl unsubstituiert oder substituiert durch einen (C₁-C₆)-Alkylrest, einen (C₁-C₅)-Alkoxyrest, durch ein Halogenatom, eine Nitrogruppe, eine Hydroxygruppe oder eine Trifluormethylgruppe bedeutet;
deren Stereoisomere, deren Additionssalze, deren Hydrate und/oder deren Solvate.

2. Verbindung der Formel (I) nach Anspruch 1, worin R₆ eine Phenylgruppe oder eine Tetrahydronaphthylgruppe darstellt, die durch einen oder mehrere Substituenten Z, wie sie für (I) definiert worden sind, substituiert ist. und R₁, R₂, R₃, R₄ und R₅ die für (I) angegebenen Bedeutungen besitzen, eines ihrer Stereosiomeren, eines ihrer Salze, eines ihrer Hydrate und/oder eines ihrer Solvate.

3. Verbindung der Formel (I) nach den Ansprüchen 1 und 2, worin R₄ eine Methylgruppe darstellt und R₁, R₂, R₃ und R₅ die für (I) angegebenen Bedeutungen besitzen, eines ihrer Stereoisomeren, eines ihrer Hydrate und/oder eines ihrer Solvate.

4. Verbindung der Formel (I) nach Anspruch 3, worin R₁ und/oder R₂ Halogen, Trifluormethyl, (C₁-C₅)-Alkyl oder (C₁-C₅)-Alkoxy, R₄ Methyl, R₆ Phenyl, das mindestens in der 2-Stellung durch einen Substituenten Z, wie er für (I) definiert worden ist, substituiert ist, bedeuten und R₃ und R₅ die für (I) angegebenen Bedeutungen besitzen, eines ihrer Stereoisomeren, eines ihrer Salze, eines ihrer Hydrate und/oder eines ihrer Solvate.

5. Verbindung nach Anspruch 1, ausgewählt aus:
. 4-(2-Chlor-4-methoxyphenyl)-5-methyl-2-[N-(5-ethoxycarbonyl-2-methoxyphenyl)-N-propylamino]-thiazol,
. 4-(2-Chlor-4-methoxyphenyl)-5-methyl-2-[N-(5-chlor-2-methylphenyl)-N-propylamino]-thiazol,
. 4-(2-Chlor-4-methoxyphenyl)-5-methyl-2-[N-(2-trifluormethylphenyl)-N-propylamino]-thiazol,
. 4-(2-Chlor-4-methoxyphenyl)-5-methyl-2-[N-(2-methoxyphenyl)-N-propylamino]-thiazol.
. 4-(2-Chlor-4-methoxyphenyl)-5-methyl-2-[N-(2,6-dichlorphenyl)-N-propylamino]-thiazol,
. 4-(2-Chlor-4-methoxyphenyl)-5-methyl-2-[N-(2,5-dichlorphenyl)-N-propylamino]-thiazol.
. 4-(2-Chlor-4-methoxyphenyl)-5-methyl-2-[N-(2-chlor-5-methylphenyl)-N-propylamino]-thiazol,
. 4-(2-Chlor-4-methoxyphenyl)-5-methyl-2-[N-(2-chlor-5-trifluormethylphenyl)-N-propylamino]-thiazol,
. 4-(2-Chlor-4-methoxyphenyl)-5-methyl-2-[N-(2-chlor-5-methoxyphenyl)-N-propylamino]-thiazol,
. 4-(2-Chlor-4-methoxyphenyl)-5-methyl-2-[N-(5-chlor-2-methoxyphenyl)-N-propylamino]-thiazol,
. 4-(2-Chlor-4-methoxyphenyl)-5-methyl-2-[N-(5-chlor-2-ethoxyphenyl)-N-propylamino]-thiazol.
. 4-(2-Chlor-4-methoxyphenyl)-5-methyl-2-[N-(2-methoxy-5-methylphenyl)-N-propylamino]-thiazol.
. 4-(2-Chlor-4-methoxyphenyl)-5-methyl-2-[N-(2,5-dimethylphenyl)-N-propylamino]-thiazol.
. 4-(2-Chlor-4-methoxyphenyl)-5-methyl-2-[N-(2,5-ditrifluormethylphenyl)-N-propylamino]-thiazol.
. 4-(2-Chlor-4-methoxyphenyl)-5-methyl-2-[N-(2-methoxy-5-trifluormethylphenyl)-N-propylamino]-thiazol,
. 4-(2-Chlor-4-methoxyphenyl)-5-methyl-2-[N-(2,5-dimethoxyphenyl)-N-propylaminol-thiazol,
. 4-(2-chlor-4-methoxyphenyl)-5-methyl-2-[N-(2-methoxy-5-methoxycarbonylphenyl)-N-propylamino]-thiazol.
. 4-(2,4-Dichlorphenyl)-5-methyl-2-[N-(2,5-dichlorphenyl)-N-propylamino]-thiazol,
. 4-(2-Chlor-4-methoxyphenyl)-5-methyl-9-[N-(5-acetyl-2-methoxyphenyl)-N-propylamino]-thiazol,
. 4-(2-Chlor-4-methoxyphenyl)-5-methyl-2-[N-(2-(methoxy)-5-(phenyl)-phenyl-N-propylamino]-thiazol.
. 4-(2-Chlor-4-methoxyphenyl)-5-methyl-2-[N-(2,6-dimethoxyphenyl)-N-propylamino]-thiazol.
. 4-(2-Chlor-4-methoxyphenyl)-5-methyl-2-[N-(2-methoxy-6-methylphenyl)-N-propylamino]-thiazol,
. 4-(2,4-Dichlorphenyl)-5-methyl-2-[N-(2-methoxy-6-methylphenyl)-N-propylamino]-thiazol,
. 4-(2-Chlor-4-methoxyphenyl)-5-methyl-2-[N-(5-ethyl-2-methoxyphenyl)-N-propylamino]-thiazol,
. 4-(2-Chlor-4-methoxyphenyl)-5-methyl-2-[N-(5-brom-2-methoxyphenyl)-N-propylamino]-thiazol,
. 4-(2-Chlor-4-methoxyphenyl)-5-methyl-2-[N-(2-methoxy-5-o-tolylphenyl)-N-propylamino]-thiazol,
. 4-[2,4,6-Trichlorphenyl)-5-methyl-2-[N-(2,5-ditrifluormethylphenyl)-N-propylamino]-thiazol,
. 4-(2,4-Dichlorphenyl)-5-methyl-2-[N-(2,5-ditrifluormethylphenyl)-N-propylamino]-thiazol,
. 4-(2-Chlor-4-methoxyphenyl)-5-methyl-2-[N-(2-methoxy-5-nitrophenyl)-N-propylaminol-thiazol,
. 4-(2,4-Dichlorphenyl)-5-methyl-2-[N-(2,6-dichlor-5-methylphenyl)-N-propylamino]-thiazol,
. 4-(2,4-Dichlorphenyl)-5-methyl-2-[N-(2-methoxy-6-methylphenyl)-N-propylamino]-thiazol,
. 4-(2,4-Dichlorphenyl)-5-methyl-2-[N-(5-chlor-2-methylphenyl)-N-propylamino]-thiazol,
. 4-(4-chlor-2-trifluormethylphenyl)-5-methyl-2-[N-(5-chlor-2-methylphenyl)-N-propylamino]-thiazol.
. 4-[2-Chlor-4-methoxy-5-methylphenyl)-5-methyl-2-[N-(2-methoxy-5-methylphenyl)-N-propylamino]-thiazol,
. 4-(2-Chlor-4-methoxyphenyl)-5-methyl-2-(N-(2-methylthio-5-trifluormethylphenyl)-N-propylamino]-thiazol.
. 4-(2,4-Dichlor-5-methylphenyl)-5-methyl-2-[N-(2-methoxy-5-methylphenyl)-N-propylamino]-thiazol.
. 4-(2-Dichlor-4,5-dimethylphenyl)-5-methyl-2-[N-(2-methoxy-5-methylphenyl)-N-propylamino]-thiazol,
. 4-(2-Chlor-4-methoxyphenyl)-5-methyl-2-[N-(2-methoxy-5-methoxycarbonylphenyl)-N-propylamino]-thiazol.
. 4-(2-Chlor-4-methoxyphenyl)-5-methyl-2-[N-(5-chlor-2-methylphenyl)-N-prop-2-inylamino]-thiazol,
eines ihrer Stereoisomeren, eines ihrer Salze, eines ihrer Hydrate und/oder eines ihrer Solvate.

6. Verfahren zur Herstellung der Verbindungen der Formel (I) nach Anspruch 1, **dadurch gekennzeichnet, daß** man ein alpha-Halogenderivat der Formel (III) 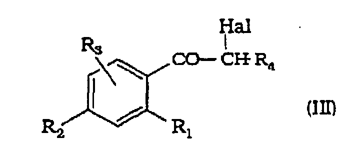 in der R₁, R₂, R₃ und R₄ die für (I) angegebenen Bedeutungen besitzen und Hal ein Halogenatom, vorzugsweise Chlor oder Brom, darstellt,
entweder mit einem Thioharnstoff (WEG A) der Formel: 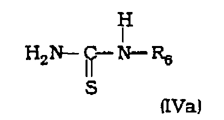 in der R₆ die für (I) angegebenen Bedeutungen besitzt, umsetzt zur Bildung einer Verbindung der Formel (II) 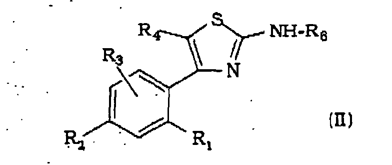 in der R₁, R₂, R₃, R₄ und R₆ die für (I) angegebenen Bedeutungen besitzen. welche man anschließend einer Alkylierungsreaktion unterwirft zur Bildung der Verbindung (I).
oder mit einem Thioharnstoff (WEG B) der Formel 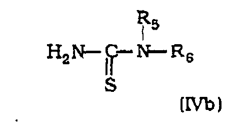 in der R₅ und R₆ die für (1) angegebenen Bedeutungen besitzen, umsetzt, zur direkten Bildung der Verbindung (I),
und man gegebenenfalls die in dieser Weise erhaltenen Verbindungen der Formel (I) anschließend gegebenenfalls in ihre mögliche Stereoisomeren trennt und/oder in ihre entsprechenden Salze überführt.

7. Verwendung der Verbindungen der Formel (I) nach einem der Ansprüche 1 bis 5 für die Herstellung eines Arzneimittels zur Behandlung von Krankheiten. welche die Modulierung der Wirkung des Freisetzungsfaktors des kortikotropen Hormons erforderlich machen.

8. Pharmazeutische Zubereitung enthaltend als Wirkstoff mindestens eine Verbindung nach einem der Ansprüche 1 bis 5 in Form der Base oder in Form des Salzes mit einer anorganischen oder organischen, pharmazeutisch annehmbaren Säure in Kombination oder in Mischung mit einern inerten, nichttoxischen, pharmazeutisch annehmbaren Trägermaterial.

9. Pharmazeutische Zubereitung nach Anspruch 8 in Form vo Dosiseinheiten. in denen der Wirkstoff mit mindestens einem pharmazcutischen Trägermaterial vermischt ist.

10. Zubereitung nach Anspruch 9, **dadurch gekennzeichnet, daß** jede Dosiseinheit 0,5 bis 800 mg des Wirkstoffs enthält.

11. Zubereitung nach Anspruch 10, Worin jede Dosiseinheit 0,5 bis 200 mg des Wirkstoffs enthält.

12. Pharmazeutische Zubereitung enthaltend eine Verbindung nach einem der Ansprüche 1 bis 5 in Kombination mit einem anderen anxiolytischen, antidepressiven oder anorexigenen Wirkstoff.

13. Verbindung nach Anspruch 1, worin R₆ eine monocyclische heteroaromatische Gruppe ausgewählt aus:
- Azepin, Pyridyl, Pyrazinyl, Pyridazinyl, Pyrimidyl, Triazinyl, Furyl und Thienyl darstellt.

14. Verbindung nach Anspruch 1, worin R₆ eine bicyclische Gruppe darstellt ausgewählt aus:
- 1,2,3,4-Tetrahydrochinolyl, 1,2,3,4-Tetrahydroisochinolyl, 1,2,3,4-Tetrahydrophthalazinyl, 1,2,3,4-Tetrahydrochinazolinyl, 1,2,3,4-Tetrahydrochinoxalinyl, 1,2,3,4-Tetrahydrocinnolinyl, 1,2,3,4-Tetrahydrobenzo-triazinyl. Chromanyl, Isochromanyl, Indolinyl. Isoindolinyl, 2,3-Dihydroindazyl, 2,3-Dihydrobenzoimidazolyl, 1,2-Dihydrobenzotriazolyl, 2,3-Dihydrobenzofuranyl, 2,3-Dihydrobenzothienyl, 2.3-Dihydrobenzosiothiazolyl, 2,3-Dihydrobenzothiazolyl, 2,3-Dihydrobenzoisoxazolyl, 2,3-Dihydrobenzoxazolyl, 1,2-Dihydrobenzoxazinyl, 1,2,3,4-Tetrahydropteridinyl, 8,9-Dihydropurinyl.
